# EUROPEAN PATENT APPLICATION

(11) **EP 4 137 559 A1**
(43) Date of publication of application: **22.02.2023**
(21) Application number: 21789510.1
(22) Date of filing: 14.04.2021
(51) Int. Cl.: C12N 5/07, C12N 15/09, C12Q 1/04, C12Q 1/68

(54) **METHOD FOR HARVESTING EXTRACELLULAR VESICLES**

(30) Priority: 15.04.2020 JP 2020073131
(71) Applicant: H.U. Group Research Institute G.K., Akiruno-shi, Tokyo 197-0833 (JP)
(72) Inventor: INUZUKA, Tatsutoshi, Akiruno-shi, Tokyo 197-0833 (JP); MORI, Akiko, Akiruno-shi, Tokyo 197-0833 (JP); KAWASAKI, Yuki, Akiruno-shi, Tokyo 197-0833 (JP); KURIMOTO, Ayako, Akiruno-shi, Tokyo 197-0833 (JP)
(74) Representative: Müller-Boré & Partner Patentanwälte PartG mbB
(86) International application number: PCT/JP2021/015500
(87) International publication number: WO 2021/210622

(57) **Abstract**

The present invention provides a method which can recover an extracellular vesicle at a high efficiency as a method alternative to conventional methods. More specifically, the present invention provides a method for recovering an extracellular vesicle, including the following steps (a) and (b):
(a) mixing (i) an extracellular vesicle-containing sample, (ii) particles on which a substance having an affinity to extracellular vesicle membrane is immobilized and (iii) a polymer to give a mixture solution containing (i') target particles bound to the extracellular vesicle via the substance and (ii') the polymer; and
(b) separating the target particles from the mixture solution. Preferably, the method further includes reducing a viscosity of the mixture solution between the above steps (a) and (b).

## Description

### FIELD

The present invention relates to a method for recovering extracellular vesicle, and the like.

### Background

Extracellular vesicles (EV) are microscopic vesicles that are secreted from various types of cells and have membrane structures, and exist in body fluids such as blood or cell culturing medium. The extracellular vesicles secreted extracellularly include exosomes, ectosomes, and apoptotic blebs. Since the extracellular vesicle are various groups that contain various substances that play a function such as intercellular signaling, it has been analyzed for the purposes such as diagnosis and drug discovery. Thus, it is required to develop a method of recovering the extracellular vesicles useful for such analyses. For example, Patent Literature 1 describes a method of recovering extracellular vesicles using a chelating agent. Patent Literature 2 describes a method for isolating extracellular vesicles by centrifuging a sample containing extracellular vesicles in the presence of two types of polymers including polyvinylpyrrolidone, centrifuging the two types of polymers to form two layers, and concentrating the extracellular vesicles at the boundary of these two layers. Patent Literature 3 describes a method for isolating extracellular vesicles by co-precipitation of extracellular vesicles with an extracellular matrix-forming polymer containing polyvinylpyrrolidone, in the presence of a polycationic substance.

### CITATION LIST

### PATENT LITERATURE

Patent Literature 1: WO2018/070479
Patent Literature 2: U.S. Patent Application Publication No. 2018/0164197 description
Patent Literature 3: WO2017/178472

### SUMMARY

### TECHNICAL PROBLEM

If extracellular vesicles can be recovered from samples containing extracellular vesicles with high efficiency, it is promising for applications such as diagnostics and drug discovery. However, the conventional methods cannot necessarily recover extracellular vesicles with high efficiency.

Therefore, an object of the present invention is to develop a method capable of recovering the extracellular vesicles at a high efficiency, as a method alternative to the conventional arts.

### SOLUTION TO PROBLEM

As a result of an extensive study, the inventors of the present invention have found that the extracellular vesicles can be recovered at a high efficiency with combination use of a polymers and particles (solid phase). More specifically, the extracellular vesicles can be recovered at high efficiency by (a) mixing (i) an extracellular vesicle-containing sample, (ii) particles on which a substance having an affinity to extracellular vesicle membrane is immobilized and (iii) a polymer to give a mixture solution containing (i') target particles bound to the extracellular vesicles via the substance and (ii') the polymer, and (b) separating the target particles from the mixture solution. The conventional method mentioned above neither describes nor suggests a method of using the polymer in combination with the particles (solid phase).

The inventors of the present invention have also found that the extracellular vesicles can be recovered at higher efficiency by reducing a viscosity of the mixture solution prior to separation of the particles from the mixture solution. Ultrasonic treatment is conventionally used to disrupt lipid bilayers (e.g., cell membranes) in operations such as homogenization. Therefore, when reducing by ultrasonic treatment the viscosity of the mixture solution containing the extracellular vesicles with lipid bilayers, it is expected that the ultrasonic treatment may cause disruption of the extracellular vesicles and consequently causing reduction of a recovery rate of the extracellular vesicles. However, the inventors of the present invention unexpectedly have found that the recovery rate of extracellular vesicles can be improved even when the mixture solution containing extracellular vesicles is subjected to the ultrasonic treatment to reduce its viscosity, and completed the present invention.

That is, the present invention is as follows.
[1] A method for recovering an extracellular vesicle, the method comprising the following steps (a) and (b):
   (a) mixing (i) an extracellular vesicle-containing sample, (ii) particles on which a substance having an affinity to extracellular vesicle membrane is immobilized and (iii) a polymer to give a mixture solution containing (i') target particles bound to the extracellular vesicle via the substance and (ii') the polymer; and
   (b) separating the target particles from the mixture solution.
[2] The method according to [1], comprising the following steps (a) to (c):
   (a) mixing (i) the extracellular vesicle-containing sample, (ii) the particles on which the substance having the affinity to the extracellular vesicle membrane is immobilized and (iii) the polymer to give the mixture solution containing (i') the target particles bound to the extracellular vesicle via the substance and (ii') the polymer;
   (b) reducing a viscosity of the mixture solution; and
   (c) separating the target particles from the solution obtained in the step (b).
[3] The method according to [1] or [2], comprising (I) washing the target particles and/or (II) releasing the extracellular vesicle from the target particles after separating the target particles.
[4] The method according to [2] or [3], wherein the viscosity is reduced by a treatment by ultrasonication or with an enzyme capable of degrading a polymer.
[5] The method according to [4], wherein the ultrasonication is dry type or water bath type.
[6] The method according to [4] or [5], wherein a frequency of the ultrasonication is in a range of 40 kHz or less or 950 kHz or more.
[7] The method according to any of [1] to [6], wherein the polymer is a polysaccharide, a protein, or a polyvinyl derivative having a carbonyl-containing hydrophilic group.
[8] The method according to [7], wherein the polysaccharide is a cellulose derivative in which a hydrogen atom of at least one hydroxy group in a cellulose is substituted with a carboxyalkyl or hydroxyalkyl.
[9] The method according to any of [1] to [8], wherein the polymer has a weight average molecular weight of 10 kDa or more.
[10] The method according to any of [1] to [9], wherein a concentration of the polymer in the mixture solution in the step (a) is 0.01 to 10.00% by weight.
[11] The method according to any of [1] to [10], wherein (iv) a chelating agent is further mixed in the step (a).
[12] The method according to any of [1] to [11], wherein the substance having the affinity to the extracellular vesicle membrane is an antibody against a tetraspanin membrane protein or an antibody against an extracellular matrix metalloproteinase inducer.
[13] The method according to any of [1] to [12], wherein the extracellular vesicle-containing sample is a fluid sample from an animal or a culture supernatant sample.
[14] A method for analyzing an extracellular vesicle, the method comprising the following steps (1) and (2):
   (1) separating an extracellular vesicle from an extracellular vesicle-containing sample by the method according to any one of any of [1] to [13]; and
   (2) analyzing the separated extracellular vesicle.
[15] A kit comprising (a) a polymer, (b) a substance having an affinity to extracellular vesicle membrane, and (c) an enzyme capable of degrading a polymer,
   wherein the substance is in a free form or in a form immobilized on particles, and
   the kit optionally further comprises particles when the substance is in a free form.
[16] The kit according to [15], wherein the polymer is a polysaccharide or a protein and the enzyme capable of degrading the polymer is a sugar degrading enzyme or a proteolytic enzyme.

### EFFECTS OF INVENTION

According to the present invention, extracellular vesicles can be recovered with higher efficiency. Thus, the present invention enables rapid preparation of desired amounts of extracellular vesicles and efficient preparation of large quantities of extracellular vesicles compared to conventional methods. In addition, the present invention enables the recovery of extracellular vesicles at a high purity.

### BRIEF DESCRIPTION OF DRAWINGS

FIG. 1 represents a western blotting of samples with a biotinylated anti-CD9 antibody, the samples obtained by an immunoprecipitation method of a serum specimen diluted with PBS, EDTA/EGTA-PBS ("ED/EG") or each of various CMC-PBS at different concentrations using an anti-CD9 antibody.
FIG. 2 represents a western blotting of samples with a biotinylated anti-CD9 antibody, the samples obtained by immunoprecipitating a serum specimen diluted with PBS or different concentrations of CMC-PBS together with an anti-CD9 antibody at 4°C overnight or at 37°C for one hour.
FIG. 3 represents results of particle count measurements of samples by nanoparticle tracking analysis, the samples obtained by an immunoprecipitation method of a serum specimen diluted with PBS, EDTA/EGTA-PBS ("ED/EG") or CMC-PBS using an anti-CD9 antibody.
FIG. 4 represents a western blotting of samples with a biotinylated anti-CD9 antibody, the samples obtained by an immunoprecipitation method, using an anti-CD9 antibody, of a specimen obtained by diluting a serum, and a plasma containing each of five kinds of anticoagulants (heparin, EDTA, citrate, ACD (acid-citrate-dextrose) and CPD (citrate-phosphate-dextrose) with PBS, CMC-PBS, EDTA/EGTA-PBS ("ED/EG") or EDTA/EGTA/CMC-PBS ("ED/EG/C").
FIG. 5A represents a western blotting of samples with an anti-tetraspanin membrane protein antibody (anti-CD63 antibody and anti-CD81 antibody), the samples obtained by an immunoprecipitation method of a serum specimen diluted with PBS, CMC-PBS, EDTA/EGTA-PBS ("ED/EG") or EDTA/EGTA/CMC-PBS ("ED/EG/C") using an anti-tetraspanin membrane protein antibody (anti-CD63 antibody and anti-CD81 antibody).
FIG. 5B represents a western blotting of samples with a biotinylated anti-CD9 antibody, the samples obtained by an immunoprecipitation method of a serum specimen diluted with PBS, CMC-PBS, EDTA/EGTA-PBS ("ED/EG") or EDTA/EGTA/CMC-PBS ("ED/EG/C") using an anti-CD147 antibody.
FIG. 6 represents a western blotting of samples with a biotinylated anti-CD9 antibody, the samples obtained by an immunoprecipitation method, using an anti-CD9 antibody, of a specimen obtained by diluting a body fluid (urine and saliva, for each of which two samples are used, which are represented as "#1" and "#2") with PBS, CMC-PBS, EDTA/EGTA-PBS ("ED/EG") or EDTA/EGTA/CMC-PBS ("ED/EG/C").
FIG. 7A represents a western blotting of samples with a biotinylated anti-CD9 antibody, the samples obtained by an immunoprecipitation method of a serum specimen diluted with PBS, each of various HEC-PBS at different concentrations, or CMC-PBS using an anti-CD9 antibody.
FIG. 7B represents a western blotting of samples with a biotinylated anti-CD9 antibody, the samples obtained by an immunoprecipitation method of a serum specimen diluted with PBS, each of various HPC-PBS at different concentrations, or CMC-PBS using an anti-CD9 antibody.
FIG. 7C represents a western blotting of samples with a biotinylated anti-CD9 antibody, the samples obtained by an immunoprecipitation method of a serum specimen diluted with PBS, each of various HPMC-PBS at different concentrations, or CMC-PBS using an anti-CD9 antibody.
FIG. 8 represents a western blotting of samples with a biotinylated anti-CD9 antibody, the samples obtained by an immunoprecipitation method of a serum specimen diluted with PBS or each of various PVP-PBS at different concentrations using an anti-CD9 antibody.
FIG. 9A represents a western blotting of samples with a biotinylated anti-CD9 antibody, the samples obtained by immunoprecipitating a serum specimen diluted with PBS or CMC-PBS together with an anti-CD9 antibody at different temperatures from 35 to 60°C.
FIG. 9B represents a western blotting of samples with an anti-CD63 antibody, the samples obtained by immunoprecipitating a serum specimen diluted with CMC-PBS together with an anti-CD63 antibody at different temperatures from 35 to 60°C.
FIG. 9C represents a western blotting of samples with an anti-CD81 antibody, the samples obtained by immunoprecipitating a serum specimen diluted with CMC-PBS together with an anti-CD81 antibody at different temperatures from 35 to 60°C.
FIG. 10 represents a detection amount (represented as fold change with reference to a sample diluted with PBS) of EML4-ALK mRNA in a sample that is diluted with PBS, CMC-PBS ("CMC") EDTA/EGTA-PBS ("ED/EG") or EDTA/EGTA/CMC-PBS ("ED/EG/C") and obtained by immunoprecipitation method of a culture supernatant from human lung carcinoma cell line H2228 using the anti-CD9 antibody or the anti-CD63 antibody.
FIG. 11 represents relative comparison of amounts of residual magnetic particles in the case of using carboxymethylcellulose (CMC). Cellulase: With cellulase treatment; Non-US: Without ultrasonic treatment. The ultrasonication frequencies applied were 100 kHz, 200 kHz, 950 kHz, and 1.6 MHz.
FIG. 12A represents relative comparison of amounts of residual magnetic particles in the case of using CMC. Non-US: Without ultrasonic treatment; US: With ultrasonic treatment (30 kHz).
FIG. 12B represents relative comparison of counts of CD9 in the case of using CMC. Non-US: Without ultrasonic treatment; US: With ultrasonic treatment (30 kHz).
FIG. 12C represents relative comparison of EV recovery efficiency based on detection of CD9 by a western blotting method with an anti-CD9 antibody for magnetic particles magnetically collected from CMC-containing solution. Non-US: Without ultrasonic treatment; US: With ultrasonic treatment (30 kHz).
FIG. 13 represents relative comparison of amounts of residual magnetic particles in the case of using CMC. Cellulase: With cellulase treatment; Non-US: Without ultrasonic treatment. Solutions comprising different final concentrations of CMC are used as the CMC-containing solutions.
FIG. 14 represents relative comparison of amounts of residual magnetic particles in the case of using cellulose derivatives other than CMC. Cellulase: With cellulase treatment; Non-US: Without ultrasonic treatment. As the cellulose derivatives, different final concentrations of hydroxypropyl cellulose 80 kDa (HPC80K) and hydroxyethyl cellulose 380 kDa (HEC380K) manufactured by Drich) are used.
FIG. 15 represents relative comparison of amounts of residual magnetic particles by different ultrasonication treatments. Cellulase: With cellulase treatment; Non-US: Without ultrasonic treatment. The frequency of the ultrasonic treatments was 30 kHz (10w, 20w or 35w output) or 40 kHz (70w output).
FIG. 16 represents relative comparison of EV recovery efficiency based on detection of CD9 by a western blotting method with an anti-CD9 antibody for magnetic particles magnetically collected after treatment with sugar degrading enzyme.
FIG. 17 represents nanoparticle tracking analysis of extracellular vesicles released from magnetic particles. Non-US: Without ultrasonic treatment; CMC: With ultrasonic treatment (using CMC); HEC: With ultrasonic treatment (using HEC).
FIG. 18 represents relative comparison of EV recovery efficiency based on detection of CD9 by a western blotting method with an anti-CD9 antibody for magnetic particles magnetically collected from cellulose derivative-containing solution. Non-US: Without ultrasonic treatment; CMC: carboxymethyl cellulose; HEC: hydroxyethyl cellulose.

### EMBODIMENTS FOR CARRYING OUT THE INVENTION

### 1. Method for recovering extracellular vesicle

The present invention provides a method of recovering an extracellular vesicle.

The extracellular vesicle is a microscopic vesicle that is secreted from various cells and has a membrane structure. Examples of the extracellular vesicle include exosomes, ectosomes and apoptotic vesicles. Preferably, the extracellular vesicle is the exosome. The extracellular vesicle can also be defined by its size. The size of the extracellular vesicle is, for example, 30 to 1000 nm, preferably 50 to 300 nm, and more preferably 80 to 200 nm. The size of the extracellular vesicle can be measured by, for example, a method based on Brownian movement of the extracellular vesicle, a light scattering method, and an electric resistance method, and the like. Preferably, the size of the extracellular vesicle is measured by NanoSight (manufactured by Malvern Instruments).

The recovery method of the present invention includes the following steps (a) and (b):
(a) mixing (i) an extracellular vesicle-containing sample, (ii) particles on which a substance having an affinity to extracellular vesicle membrane is immobilized and (iii) a polymer to give a mixture solution containing (i') target particles bound to the extracellular vesicles via the substance and (ii') the polymer; and
(b) separating the target particles from the mixture solution.

At the above step (a), (i) the sample containing the extracellular vesicles, (ii) the particles on which the substance having the affinity to the extracellular vesicle membrane is immobilized and (iii) the polymer are mixed. With this mixing, the extracellular vesicles in the sample (i) is bound to the substance having the affinity to the extracellular vesicle membrane that is immobilized on the particles (ii) to form the target particles that are bound to the extracellular vesicles via the substance. Thus, the mixture solution that contains (i') the target particles bound to the extracellular vesicles via the substance having affinity to the extracellular vesicle membrane and (ii') the polymer is obtained.

At the above step (a), the mixing of (i) to (iii) is not particularly limited as long as the mixture solution containing (i') the target particles bound to the extracellular vesicles via the substance and (ii') the polymer is obtained. For example, the mixing of (i) to (iii) can be performed simultaneously or separately. When the mixing of (i) to (iii) is performed separately, two of (i) to (iii) may be mixed, and then the resulting mixture may be added to the remaining one for the mixing. Alternatively, two of (i)-(iii) may be added to the remaining one for the mixing. Preferably, (i) and (ii) may be added to (iii) for the mixing. For example, the mixing can be performed by inversion mixing or stirring.

The extracellular vesicle containing sample of (i) is any sample containing extracellular vesicles. Preferably, the extracellular vesicle-containing sample is a biological liquid sample. The extracellular vesicle-containing sample may be subjected to another treatment before used in the method of the present invention. Examples of such a treatment include centrifugal separation, extraction, filtration, precipitation, heating, freezing, refrigeration, and agitation.

In one embodiment, the extracellular vesicle-containing sample is a culture supernatant sample. The culture supernatant sample may be a cell culture supernatant sample or a tissue culture supernatant sample. Examples of the organism from which a cell or a tissue to be cultured is derived include animals such as mammalian animals (e.g., primates such as humans and monkeys; rodents such as mice, rats and rabbits; farm animals such as cattle, pigs and goats; and working animals such as horses and sheep) and birds (e.g., chickens), insects, microorganisms (e.g., bacteria), plants, and fish. The organism is preferably the mammalian animal and preferably human.

In another embodiment, the extracellular vesicle-containing sample is an animal-derived liquid sample. The animal-derived liquid sample is a body fluid from the organism as described above. Examples of the body fluid include blood samples (e.g., whole blood, serum and plasma), urine, saliva, lymph fluid, tissue fluid, cerebrospinal fluid, ascites, sweat, seminal fluid, tear fluid, mucosal fluid, milk, thoracic fluid, bronchoalveolar lavage fluid and amnion fluid. Preferably, the body fluid is blood sample, urine or saliva. Examples of the plasma include heparin plasma, citrate plasma, sodium fluoride plasma, and a plasma that contains acid-citrate-dextrose (ACD) or citrate phosphate dextrose (CPD). In general, compared to the culture supernatant, it is difficult to recover extracellular vesicles in a body fluid (e.g., blood, urine and saliva) that contains more proteins (e.g., albumin, lysozyme, lactoferrin, histatin, peroxidase, agglutinin, defensin and immunoglobulin) than the culture supernatant. In contrast, the method of present invention can recover the extracellular vesicles with high purity at a high efficiency even from such a body fluid since an amount of the extracellular vesicles to be recovered from the extracellular vesicle-containing sample is increased.

As the particles (ii), it is possible to use any particles on which a substance having affinity to the extracellular vesicle membrane is immobilized. The particles are not particularly limited as long as they can immobilize the substance having affinity to the extracellular vesicle membrane and can be collected (e.g., by magnetic manipulation or centrifugal separation) from the mixture solution obtained in the above step (a). Examples of the particles include microparticles, nanoparticles, microbeads, nanobeads, microspheres, and nanospheres. Examples of the particles also include inorganic particles (e.g., metal particles and silica particles), organic particles (e.g., polymer particles), and organic-inorganic composite particles. The particles may be spherical or non-spherical (e.g., oval) shape.

The average primary particle size of the particles is not particularly limited. From the viewpoint of ease of collection, it may be, for example, 0.001 to 1000 µm, preferably 0.01 to 100 µm, more preferably 0.1 to 10 um, still more preferably 0.5 to 5 µm, and particularly preferably 1 to 3 µm. The average primary particle size of the particles can be measured by the BET method.

Preferably, the particles are magnetic particles from the viewpoint of ease of the collection by magnetic manipulation. The magnetic particles are particles containing magnetic materials such as iron, nickel, cobalt, or an alloy thereof (e.g., ferrite). The magnetic particles preferably have an average primary particle size of 1 to 3 µm.

The substance having an affinity to extracellular vesicle membranes, which is immobilized to the particles, is a substance having an ability to bind to a surface marker of an extracellular vesicle. Examples of the surface marker of an extracellular vesicle include a tetraspanin membrane protein (extracellular vesicle membrane-specific four times transmembrane membrane proteins, e.g., CD9, CD63 and CD81), an extracellular matrix metalloproteinase inducer (e.g., CD147), heat shock protein (HSP) 70, HSP90, major histocompatible complex (MHC) I, lysosome associated membrane protein (LAMP) 1, intercellular adhesion molecule (ICAM)-1, integrin, ceramide, cholesterol, phosphatidylserine, Annexins, Caveolin-I and EpCAM. The surface markers of extracellular vesicles are preferably tetraspanin membrane proteins (e.g., CD9 and CD63) or extracellular matrix metalloproteinase inducers (e.g., CD81 or CD147).

Examples of the substance having an affinity to the extracellular vesicle membrane include antibodies, aptamers, phosphatidylserine-bound proteins and ceramide-bound proteins to the surface marker of the extracellular vesicle. In the present invention, for the substance having an affinity to the surface marker of the extracellular vesicle, single substance or a plurality of types (e.g., two, three, or four types) of substances can be used.

Preferably, the substance having an affinity to the surface marker of the extracellular vesicle may be an antibody to the surface marker of the extracellular vesicle, from the viewpoints such as those of ensuring specificity to the surface marker of the extracellular vesicle and simplicity of preparation. Examples of the antibody include full-length antibodies (e.g., monoclonal antibodies and polyclonal antibodies) and antigen-binding fragments thereof. The antigen-binding fragment may be antibody fragment that maintains capability of binding to the targeted EV surface marker, and include Fab, Fab', F(ab')₂, scFv or the like. A single antibody or a plurality kinds (e.g., two, three, or four kinds) of antibodies can be used in the present invention.

As the polymer (iii), the polymer may be used as it is, and it is preferable to use an aqueous polymer solution in which the polymer is dissolved in an aqueous solution (e.g., buffer solution) in order to reduce viscosity for facilitating the mixing. The polymer concentration in the aqueous polymer solution differs depending on factors such as kinds of the polymer and degree of polymerization, and may be, for example, 0.01 to 30% by weight, preferably 0.02 to 25% by weight, more preferably 0.05 to 20% by weight, still more preferably 0.1 to 15% by weight, and particularly preferably 0.2 to 10% by weight. Preferably, the polymer may be a water soluble polymer. The water soluble polymer refers to a polymer having a solubility of 0.01% by weight or more in water at a temperature of 4 to 80°C (preferably 4 to 37°C). The solubility of the water soluble polymer in water at 4 to 80°C (preferably 4 to 37°C) may be preferably 0.05% by weight or more and more preferably 0.1% by weight or more.

In order to accomplish the object of the present invention, the polymer may have a value of 1.5 mPa-s or more as a viscosity in an aqueous solution with 1 to 20% by weight at 20 to 30°C, for example. The viscosity of the polymer under the above condition may be preferably 5 mPa·s or more, more preferably 10 mPa·s or more, still more preferably 20 mPa·s or more, still further more preferably 30 mPa·s or more, and particularly preferably 35 mPa·s or more. The viscosity of the polymer under the above condition may be preferably 30000 mPa·s or less, more preferably 20000 mPa·s or less, still more preferably 10000 mPa·s or less, still further more preferably 5000 mPa·s or less, and particularly preferably 1000 mPa·s or less. More specifically, the viscosity of the polymer under the above condition may be preferably 5 to 30000 mPa·s, more preferably 10 to 20000 mPa·s, still more preferably 20 to 10000 mPa·s, still further more preferably 30 to 5000 mPa·s, and particularly preferably 35 to 1000 mPa·s.

In order to accomplish the object of the present invention, the polymer may have a value of 1.5 mPa-s or more as a viscosity at 30°C in a phosphate-buffered saline (PBS) solution that is prepared by dissolving the polymer in the PBS to achieve 2% by weight, for example. The viscosity of the polymer under the above condition may be preferably 5 mPa·s or more, more preferably 10 mPa-s or more, still more preferably 20 mPa·s or more, still further more preferably 30 mPa·s or more, and particularly preferably 35 mPa-s or more. The viscosity of the polymer under the above condition may be preferably 30000 mPa·s or less, more preferably 20000 mPa·s or less, still more preferably 10000 mPa·s or less, still further more preferably 5000 mPa·s or less, and particularly preferably 1000 mPa-s or less. More specifically, the viscosity of the polymer under the above condition may be preferably 5 to 30000 mPa·s, more preferably 10 to 20000 mPa-s, still more preferably 20 to 10000 mPa·s, still further more preferably 30 to 5000 mPa·s, and particularly preferably 35 to 1000 mPa·s.

The viscosity of the polymer can be measured by either a method of detecting a viscosity friction torque of liquid generated at an exterior periphery of a rotor while a liquid sample is rotated by the rotor (method of using a rotary viscometer), or a method of measuring a falling time of a falling weight while the falling weight is falling freely inside a measurement tube filled with the sample (method of using falling ball viscometer). In addition, the vibration amplitude of the sensor is suppressed with an increase in a liquid viscosity due to its viscosity resistance in a case such as the case of putting oscillator (viscosity sensor) inside the liquid sample and vibrating it. Then, the viscosity of the polymer can be measured by a method of measuring an amount of input electric current while an oscillator driving current is increased so as to maintain a constant vibration amplitude by overcoming a force that suppresses the vibration amplitude of the sensor (method of using vibration viscometer). The viscosity of the polymer can be preferably measured with a viscosity analyzer (e.g., Rheology Spectrometer SKR100 manufactured by Yamato Scientific Co., Ltd.).

Furthermore, the polymer may have a weight-average molecular weight of 10 kDa or more, for example, in order to accomplish the object of the present invention. The weight-average molecular weight of the polymer may be preferably 12 kDa or more, more preferably 14 kDa or more, still more preferably 16 kDa or more, still further more preferably 18 kDa or more, and particularly preferably 20 kDa or more. The weight-average molecular weight of the polymer may be preferably 5000 kDa or less, more preferably 3000 kDa or less, still more preferably 2000 kDa or less, still further more preferably 1000 kDa or less, and particularly preferably 500 kDa or less. More specifically, the weight-average molecular weight of the polymer may be preferably 12 to 5000 kDa, more preferably 14 to 3000 kDa, still more preferably 16 to 2000 kDa, still further more preferably 18 to 1000 kDa, and particularly preferably 20 to 500 kDa.

Any polymer with the properties described above can be used as the polymer. Examples of such a polymer include polysaccharides, proteins, polyether compounds, and polyvinyl derivatives with hydrophilic groups.

The polysaccharide is a saccharide polymer. Examples of the polysaccharide include simple polysaccharides (e.g., cellulose, cellulose derivatives, starch, and glycogen) and complex polysaccharides (e.g., hyaluronic acid, chondroitin sulfate, and heparin). The polysaccharide is preferably the cellulose derivative from the viewpoints of improvements of efficiency of extracellular vesicle recovery, water solubility, and ease of availability.

The cellulose derivative is a cellulose derivative in which a hydrogen atom in at least one hydroxy group of the cellulose is substituted with a hydrophilic group. Examples of the hydrophilic group in the cellulose derivative include carboxyalkyls (e.g., carboxy C₁₋₆ alkyl) and hydroxyalkyls (e.g., hydroxy C₁₋₆ alkyl). The hydrophilic group in the cellulose derivative is preferably carboxyalkyls or hydroxyalkyls.

Examples of the carboxyalkyl include carboxymethyl, carboxyethyl (1-carboxyethyl and 2-carboxyethyl), carboxypropyl (1-carboxypropyl, 2-carboxypropyl_ and 3-carboxypropyl), carboxyisopropyl (1-carboxy-2-methylethyl and 2-carboxy-2-methylethyl), carboxybutyl (1-carboxybutyl, 2-carboxybutyl, 3-carboxybutyl and 4-carboxybutyl), carboxy t-butyl, carboxypentyl (1-carboxypentyl, 2-carboxypentyl, 3-carboxypentyl, 4-carboxypentyl and 5-carboxypentyl), carboxyhexyl (1-carboxyhexyl, 2-carboxyhexyl, 3-carboxyhexyl, 4-carboxyhexyl, 5-carboxyhexyl and 6-carboxyhexyl).

Examples of the hydroxyalkyl include hydroxymethyl, hydroxyethyl (1-hydroxyethyl and 2-hydroxyethyl), hydroxypropyl (1-hydroxypropyl, 2-hydroxypropyl and 3-hydroxypropyl), hydroxyisopropyl (1-hydroxy-2-methylethyl and 2-hydroxy-2-methylethyl), hydroxybutyl (1-hydroxybutyl, 2-hydroxybutyl, 3-hydroxybutyl and 4-hydroxybutyl), hydroxy t-butyl, hydroxypentyl (1-hydroxypentyl, 2-hydroxypentyl, 3-hydroxypentyl, 4-hydroxypentyl and 5-hydroxypentyl) and hydroxyhexyl (1-hydroxyhexyl, 2-hydroxyhexyl, 3-hydroxyhexyl, 4-hydroxyhexyl, 5-hydroxyhexyl and 6-hydroxyhexyl).

Specific examples of the cellulose derivative include carboxymethyl cellulose (CMC), hydroxyethyl cellulose (HEC), hydroxypropyl cellulose (HPC) or hydroxypropylmethyl cellulose (HPMC).

The examples of cellulose derivative also include a nanocellulose derivative. The nanocellulose derivative is a derivative of a nanocellulose. The nanocellulose is a fibrous cellulose having a fiber width in a nanometer order. The fiber width of the nanocellulose is, for example, 500 nm or less, and may be preferably 200 nm or less, more preferably 100 nm or less, still more preferably 50 nm or less, still further more preferably 10 nm or less, and particularly preferably 5 nm or less.

Protein is an amino acid polymer also called as polypeptide. Examples of the protein include gelatin, casein, albumin, collagen, and alginic acid.

The polyether compound refers to a polymer containing an ether structure in a main chain of a repeat unit. Examples of the polyether compound include a polyalkyleneoxy compound (e.g., poly C₁₋₆ alkyleneoxy compound). Examples of the polyalkyleneoxy compound include polyethylene glycol and polypropylene glycol. The polyalkyleneoxy compound is preferably polyethylene glycol.

The polyvinyl derivative having a hydrophilic group refers to a polyvinyl derivative in which at least one hydrogen atom is substituted with a hydrophilic group, and is preferably a polyvinyl derivative in which at least one hydrogen atom in a methylene unit is substituted with a hydrophilic group. Examples of the hydrophilic group in the polyvinyl derivative include a carbonyl-containing hydrophilic group, a carboxy-containing hydrophilic group, a nitrogen-containing hydrophilic group, and a ring (carbocycle or heterocycle)-containing hydrophilic group. The hydrophilic group in the polyvinyl derivative is preferably the carbonyl-containing hydrophilic group, the nitrogen-containing hydrophilic group, or the heterocycle-containing hydrophilic group, and more preferably a lactam (e.g., α-lactam, β-lactam, γ-lactam, 6-lactam, and ε-lactam). Specific examples of the polyvinyl derivative having the hydrophilic group include polyvinylpyrrolidone.

Polymers such as the polysaccharide, the protein, the polyvinyl derivative with a hydrophilic group and the polyether compound also include salts thereof. Examples of the salt includes salts of metals (e.g., monovalent metals such as lithium, sodium, potassium, rubidium, and cesium; and bivalent metals such as calcium, magnesium, and zinc), and salts of inorganic base (e.g., ammonia).

Preferably, the polymer is the polysaccharide, protein, or the polyether compound, more preferably the polysaccharide or protein, still more preferably the polysaccharide, and particularly preferably the cellulose derivative.

The polymer concentration in the mixture solution is not particularly limited, as long as it allows the extracellular vesicles to be recovered at a higher efficiency than the solution without containing polymer and it allows the polymer to be dissolved in the mixture solution. Such a concentration differs depending on kinds of polymers, and may be, for example, 0.01 to 10.00% by weight, preferably 0.05 to 7.50% by weight and more preferably 0.10 to 5.00% by weight.

The mixing in the above step (a) is performed under a condition sufficient to produce the target particles that are bound to the extracellular vesicles via the substance having the affinity to the extracellular vesicle membrane. Such a temperature condition is, for example, 4 to 60°C, preferably 15 to 50°C and more preferably 20 to 45°C. The time required to prepare the mixture solution is, for example, 30 seconds or less, preferably 20 seconds or less and more preferably 15 seconds or less. The mixing under such a condition enables to increase the amount of the target particles bound to the extracellular vesicles.

The method of the present invention may further include incubating the mixture solution after the mixing in the above step (a). The incubation time differs depending on factors such as the time required to prepare the mixture, the desired recovery yield of the extracellular vesicles and the incubation temperature, and is, for example, 48 hours or less, preferably 24 hours or less, more preferably 120 minutes or less, and still more preferably 60 minutes or less. From the viewpoint of quick processing and the like, the incubation time may be still further more preferably 30 minutes or less, and particularly preferably 20 minutes or less, 10 minutes or less, or 5 minutes or less. The incubation temperature is the same as the temperature conditions in the mixing described above.

In the above step (b), the target particles are separated from the mixture solution obtained in the above step (a). The target particles are bound to the extracellular vesicle via the substance having the affinity to the extracellular vesicle membrane. Thus, the separation of target particles enables to separate the extracellular vesicles bound to the target particles.

The target particles can be separated from the mixture solution by any method. This separation can achieve so-called B (bound) / F (free) separation, in which a factor immobilized on a solid phase (target particle) is separated from a factor not immobilized on the solid phase (target particle). For example, the target particles can be separated from the mixture solution by centrifuging the mixture solution obtained in the step (a) and then removing the supernatant. When the magnetic particles are used in the step (a), the target particles can be separated from the mixture solution obtained in the step (a) by collecting the target particles by magnetic collection.

In a preferred embodiment, the method of the present invention may further include reducing the viscosity of the mixture solution obtained in the above step (a). In other words, the method of the present invention may include the following (a) to (c) :
(a) mixing (i) the extracellular vesicle-containing sample, (ii) the particles on which the substance having the affinity to the extracellular vesicle membrane is immobilized and (iii) the polymer to give the mixture solution containing (i') the target particles bound to the extracellular vesicles via the substance and (ii') the polymer;
(b) reducing the viscosity of the mixture solution; and
(c) separating the target particles from the solution obtained in the above (b).

The steps (a) and (c) in this embodiment can be performed in the same way as in the steps (a) and (b) in the method mentioned above.

In the above preferred embodiment, the step (b) can be performed by any method which can reduce the viscosity of the mixture solution obtained in the above step (a). From the viewpoint of convenience of implementation and the like, this step (b) is preferably performed by a treatment by ultrasonication or with an enzyme capable of degrading the polymer.

The treatment by ultrasonication can be performed with a dry type or water bath type. The treatment by dry type ultrasonication refers to a direct ultrasonic treatment that can be achieved by bringing the ultrasonic generating part in direct or indirect contact with a container (e.g., tube) containing the target solution (e.g., the mixture solution obtained in (a) in the present invention) without use of a water bath or the like. The treatment by water-bath type ultrasonication refers to an indirect ultrasonic treatment that can be achieved by immersing a container (e.g., tube) containing the target solution in the water bath of an ultrasonic generator. The ultrasonic treatment time is not particularly limited as long as the viscosity of the mixture solution can be reduced, and differs depending on factors such as ultrasonic conditions, and is, for example, 10 seconds to 30 minutes, preferably 20 seconds to 20 minutes, and more preferably 30 seconds to 10 minutes.

The frequency (Hz) and output (w) of the ultrasonication are not particularly limited as long as they can reduce the viscosity of the mixture solution to eventually facilitate the step (c) involving the separation of the target particles from the solution obtained in the above step (b). Such a frequency is, for example, 5 kHz to 5.0 MHz. The frequency may be preferably 10 kHz to 3.0 MHz, more preferably 20 kHz to 2.5 MHz, and still more preferably 30 kHz to 2.0 MHz, in the viewpoint of a frequency range wide enough to employ any ultrasonication generating transducer or generator. The output ranges from, for example, 5 to 300 w. The output may preferably be 10 to 200 w from the same viewpoint as the frequency.

In a certain embodiment, the frequency of the ultrasonication (Hz) may be set to remarkably improve the efficiency of recovering the extracellular vesicles bound to the target particles. Such a frequency may be in a range of, for example, 40 kHz or less (preferably 10 kHz to 40 kHz, more preferably 20 kHz to 40 kHz, and still more preferably 30 kHz to 40 kHz), or in a range of 950 kHz or more (preferably 950 kHz to 3.0 MHz, more preferably 950 kHz to 2.5 MHz, and still more preferably 950 kHz to 2.0 MHz) .

The treatment with the enzyme capable of degrading the polymer can be performed by appropriately selecting the enzyme capable of degrading the relevant polymer depending on kind of the polymer used in the above step (a).

For example, a sugar degrading enzyme can be used when the polymer used in (a) is the polysaccharide such as the cellulose derivative. Examples of the sugar degrading enzyme include cellulase, glycosidase, xylanase, lactase, amylase, chitinase, sucrase, maltase, neuraminidase, invertase, hyaluronidase and lysozyme. In addition, endo-type enzymes or exo-type enzymes can be used as the sugar degrading enzymes. From the viewpoint of efficient cleavage of the polymer in a short time leading to efficient reduction of the viscosity, the sugar degrading enzyme is preferably the endo-type enzyme. The sugar degrading enzyme can be selected depending on the kind of polysaccharide. For example, when the polymer used in (a) is the cellulose derivative, it is possible to use the sugar degrading enzyme capable of degrading the cellulose derivative such as the cellulase, an endoglucanase.

A proteolytic enzyme can be used when the polymer used in (b) is a protein. Examples of the proteolytic enzyme include aspartate protease, serine protease, cysteine protease, and metalloprotease. In addition, it is possible to use an endoprotease (e.g., trypsin, chymotrypsin, elastase, and collagenase) or an exoprotease (e.g., leucine aminopeptidase, carboxypeptidase, and dipeptidyl aminopeptidase) as the proteolytic enzyme. From the viewpoint of efficient cleavage of the polymer in a short time leading to efficient reduction of the viscosity, the proteolytic enzyme may preferably be the endoprotease.

When the substance having an affinity to the extracellular vesicle membrane used in the present invention is a substance capable of binding to a surface marker protein of the extracellular vesicle, from the viewpoint of preventing degradation of the surface marker protein, the enzyme capable of degrading the polymer is preferably the sugar degrading enzyme.

As the condition for the treatment with the enzyme capable of degrading the polymer, it is possible to employ a typical condition for enzymatic reactions (e.g., 25 to 40°C for 1 to 60 minutes), for example.

The polymer used in the present invention may be selected as appropriate according to the type of treatment used to reduce the viscosity of the mixture. For example, when the treatment to reduce the viscosity of the mixture is ultrasonic treatment, it is possible to select as appropriate the polysaccharide, the protein, the polyvinyl derivative with hydrophilic groups, or the polyether compound, for example, as the polymer. On the other hand, when the treatment to reduce the viscosity of the mixture solution is the treatment with the enzyme capable of degrading the polymer, the polymer is preferably the polysaccharide (preferably the cellulose derivative) or the protein, from the viewpoint of facilitating availability of such an enzyme, and the like. When the substance having an affinity to the extracellular vesicle membrane used in the present invention is a substance capable of binding to the surface marker protein of the extracellular vesicles, the polymer is preferably the polysaccharide from the viewpoint of preventing degradation of the surface marker protein.

The method of the present invention may use a chelating agent in combination in the mixing in the above step (a) to improve a recovery rate of the extracellular vesicles. The treatment of the extracellular vesicle-containing sample with the chelating agent can improve the recovery rate of the extracellular vesicles (e.g., WO2018/070479).

The chelating agent is a compound that has a coordination moiety capable of forming a coordination bond with a metal ion, or a salt thereof. The number of coordination moiety is preferably two or more and more preferably three or more (e.g., three or six). Examples of a coordination atom as the coordination moiety include oxygen atom, phosphorus atom, nitrogen atom, sulfur atom, and chlorine atom. The coordination atom is preferably oxygen atom or phosphorus atom and more preferably oxygen atom. Examples of a coordination group as the coordination moiety include a group having the coordination atom mentioned above. The coordination group is preferably a carboxylic acid group or a phosphate group and more preferably a carboxylic acid group.

Examples of the chelating agent include hydroxyethyl iminodiacetic acid (HIDA), nitrilotriacetic acid (NTA), hydroxyethyl ethylenediaminetriacetic acid (HEDTA), ethylenediaminetetraacetic acid (EDTA), ethylenediaminetetra(methylene phosphonic acid) (EDTMP), and glycol ether diamine tetraacetic acid (EGTA), and salts thereof. Examples of the salt include metal salts (e.g., monovalent metal salts such as sodium salts and potassium salts, and divalent metal salts such as calcium salts and magnesium salts), inorganic salts (e.g., halide salts such as fluoride, chloride, bromide, and iodide, and ammonium salts), organic salts (e.g., ammonium salts substituted with alkyl groups), and acid addition salts (e.g., salts with inorganic acids such as sulfuric acid, hydrochloric acid, hydrobromic acid, nitric acid, and phosphoric acid, and salts of organic acids such as acetic acid, oxalic acid, lactic acid, citric acid, trifluoromethanesulfonic acid, and trifluoroacetic acid).

In certain embodiments, the chelating agent may be a chelating agent that is commonly used as a component in a blood collection tube for clinical testing. Examples of such a chelating agent include EDTA, EGTA, NTA, HEDTA, EDTMP, HIDA, citric acid and salts thereof. In the present invention, the use of such a chelating agent is also desirable from the viewpoint of clinical application.

In the present invention, one chelating agent may be used alone, or a plurality of kinds of the chelating agents (e.g., two, three, or four types) may be used in combination. The concentration of the chelating agent differs depending on factors such as the type and concentration of another component used in combination with the chelating agent, and may be, for example, 10 mM to 1000 mM.

The method may further include (I) washing the target particles and/or (II) releasing the extracellular vesicle from the target particles, after the separation of the target particles bound to the extracellular vesicle via the substance having the affinity to the extracellular vesicle membrane.

The target particles can be washed using an aqueous solution (e.g., buffer solution). The number of washing is typically one to three.

The release of the extracellular vesicles from the target particles can be performed by any method capable of cleaving a binding between the extracellular vesicle and the substance having an affinity to the extracellular vesicle membrane. Examples of such a method include treatment with acid or alkali and heat treatment.

### 2. Method for analyzing extracellular vesicle

The present invention also provides a method for analyzing the extracellular vesicle. The method includes the following steps (1) and (2).
(1) separating the extracellular vesicles from the extracellular vesicle-containing sample; and
(2) analyzing the separated extracellular vesicles.

The step (1) in the analysis method of the present invention can be performed in the same way as in the method of recovering the extracellular vesicle of the present invention.

Examples of an analyte in the analysis of the extracellular vesicle in the step (2) include components contained in the extracellular vesicle (e.g., components contained inside the extracellular vesicle, membrane components of the extracellular vesicle, and components present on the membrane surface of the extracellular vesicle) and extracellular vesicle itself.

The component contained in the extracellular vesicle can be analyzed qualitatively or quantitatively. Also, such an analysis refers to an analysis of one component or plural components. Examples of the component to be analyzed include proteins, nucleic acids (e.g., RNA and DNA), saccharides, lipids, amino acids, vitamins, polyamines and peptides. The present invention can analyze the components in the extracellular vesicle with high accuracy since the present invention increase the recovery yield of the extracellular vesicles.

The component can be analyzed by any method.

In the case that the component to be analyzed is a protein, examples of the analysis method include immunoassay and mass spectrometry. Examples of the immunoassay include a direct competitive method, an indirect competitive method and a sandwich method. In addition, examples of such an immunoassay include chemiluminescent immunoassay (CLIA) [e.g., a chemiluminescent enzyme immunoassay (CLEIA)], turbidimetric immunoassay (TIA), enzyme immunoassay (EIA) (e.g., direct competitive ELISA, indirect competitive ELISA, and sandwich ELISA), radioimmunoassay (RIA), latex agglutination method, fluorescence immunoassay (FIA), and immunochromatography, Western blotting, immunostaining, and fluorescence activated cell sorting (FACS). In the case that a plurality of components are analyzed, proteomic analysis may be performed.

In the case that the component to be analyzed is the nucleic acid, examples of the analysis method include hybridization methods using probes, gene amplification methods using primer (e.g., 2, 3 or 4 primers), and mass spectrometry.

In the case that the component to be analyzed is a component other than proteins or nucleic acids, examples of the analysis method include immunoassay and mass spectrometry. When a plurality of components are analyzed, metabolome analysis may be performed.

The extracellular vesicle itself can also be analyzed qualitatively or quantitatively. For example, the extracellular vesicle can be analyzed with instruments such as particle analysis equipment, electron microscope and flow cytometer. In this case, it is possible to analyze the number, dimension, and shape of particles of the extracellular vesicle and distribution thereof.

It has been reported that the extracellular vesicles can be involved in various diseases such as cancer (WO2014/003053; WO 2014/152622; Taylor et al., Gynecologic Oncol, 100 (2008), pp. 13-21). Thus, the present invention is useful, for example, for diagnosis based on the extracellular vesicles and drug discovery.

### 3. Kit

The present invention also provides a kit for use in the method of the present invention described above.

The kit of the present invention includes the following (a) to (c):
(a) the polymer;
(b) the substance having an affinity to the extracellular vesicle membrane; and
(c) the enzyme capable of degrading the polymer.

In the kit of the present invention, the substance having an affinity to the extracellular vesicle membrane is in a free form or in a form immobilized on the particles. When the substance having an affinity to the extracellular vesicle membrane is in a free form, the kit of the present invention may further contain particles. The kit of the present invention may further include a chelating agent. Regarding definitions, examples and preferred examples of the polymer, the substance having an affinity to the extracellular vesicle membrane, the enzyme capable of degrading the polymer and the chelating agent described above in the methods of the invention, the same is applied to those in the kit. The kit of the present invention is useful for convenient and prompt implementation of the method of the present invention.

### EXAMPLES

Hereinafter, the present invention will be described with reference to Examples, but the present invention is not limited to these Examples.

### (Example 1)

The effect on EV recovery was investigated for three kinds of commercially available CMC sodium salts (hereinafter referred to simply as "CMC". CAS No. 9004-32-4, Nacalai Tesque, Inc. #07326-95 (unknown average molecular weight); Sigma-Aldrich Co. LLC #C5678 (average molecular weight 90 kDa); #C4888 (average molecular weight 250 kDa)). EV recovery was performed using the anti-CD9 antibody.

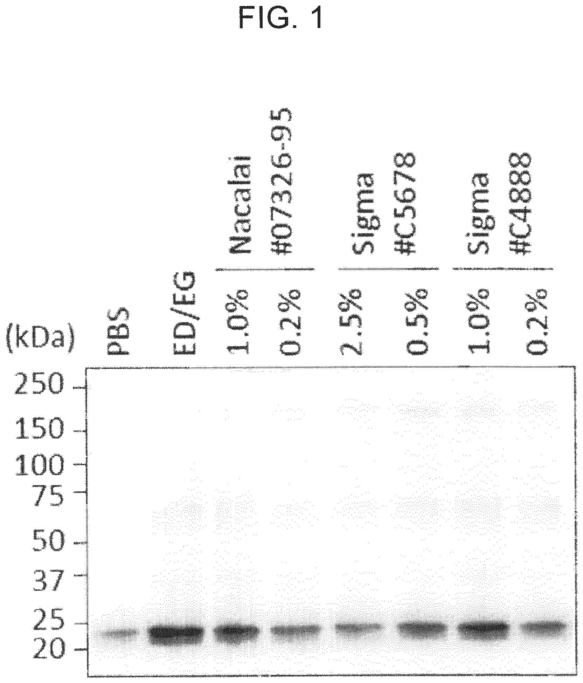

Healthy human serum was centrifuged at 20,000×g at 4°C for 15 minutes, then 200 µL of the serum was diluted with 200 µL of PBS (2.9 mM NaH₂PO₄, 9.0 mM Na₂HPO₄, 137 mM NaCl), EDTA/EGTA-PBS (PBS containing EDTA and EGTA with a final concentration of 50 mM after the dilution of the serum, respectively) (ED/EG), or CMC-PBS with a final concentration of 0.2 to 2.5% by weight (PBS in which CMC was dissolved to have a final concentration of 0.2 to 2.5% by weight after the dilution of the serum). Then, magnetic particles (Dynabeads M-280 tosylactivated (Life Technologies)) on which an anti-CD9 antibody (our product) was immobilized was added to the resultant solution to achieve 0.26 mg/mL. After the resultant solution underwent reaction at 4°C while stirred overnight, the magnetic particles were separated magnetically from the remaining solution (B/F separation). The separated magnetic particles were washed three times with PBS-T, then diluted with a sample buffer (Bio-Rad Laboratories, Inc.) for preparation of a western blotting sample. In the western blotting sample, a sample containing exosomes was treated with SDS to disrupt the exosomes for releasing a marker protein (e.g., CD9) of the exosomes in the sample solution. Immunoprecipitation efficiency was analyzed by western blotting method with use of a biotinylated anti-CD9 antibody (our product) (FIG. 1). Improvement of EV recovery efficiency was recognized for all of three CMC, compared to PBS-diluted samples.

Physical properties of CMC used in the Example are summarized in Table 1 below.

**Table 1. List of CMC used in Example**

| Product code | Manufacturer | Average molecular weight (kDa) | Viscosity / Measurement condition (Value disclosed by Manufacturer) |
|---|---|---|---|
| #07326-95 | Nacalai Tesque, Inc. | No data | No data |
| #C5678 | Sigma-Aldrich Co. LLC | Average Mw 90 | 50-200 mPa·s/ 4 wt% aqueous solution (25°C) |
| #C4888 | Sigma-Aldrich Co. LLC | Average Mw 250 | 400~800mPa·s/ 2 wt% aqueous solution (25°C) (lit.) |

### (Example 2)

The effect on EV recovery was investigated for different concentrations (with a final concentration of 0.06% by weight to 1.0% by weight) of CMC (Sigma-Aldrich Co. LLC #C4888) at different reaction temperatures (4°C and 37°C).

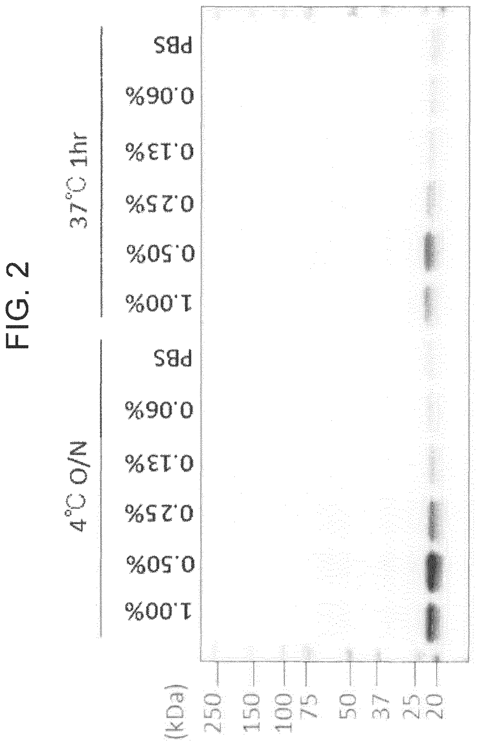

Healthy human serum was centrifuged at 20,000×g at 4°C for 15 minutes, then 200 µL of the serum was diluted with 200 µL of PBS or CMC-PBS (PBS in which CMC was dissolved to have a final concentration of 0.06 to 1.0% by weight after the dilution of the serum). Then, the magnetic particles (Dynabeads M-280 tosylactivated (Life Technologies)) on which the anti-CD9 antibody (our product) was immobilized were added to the resultant solution to achieve 0.26 mg/mL. After the resultant solution underwent reaction while stirred at 4°C overnight or at 37°C for 1 hour, the magnetic particles were separated magnetically from the remaining solution (B/F separation). The separated magnetic particles were washed three times with PBS-T, then diluted with the sample buffer (Bio-Rad Laboratories, Inc.) for preparation of the western blotting sample. Recovery efficiency of EV was analyzed by western blotting method with use of the biotinylated anti-CD9 antibody (FIG. 2). Improvement of EV recovery efficiency was recognized in the CMC final concentration range of 0.25% by weight to 1% by weight at the reaction temperatures of 4°C and 37°C, compared to the PBS-diluted samples.

### (Example 3)

The effect on EV recovery was investigated for CMC (Sigma-Aldrich Co. LLC #C4888) by nanoparticle tracking analysis (NanoSight LM10, Quantum Design, Inc.).

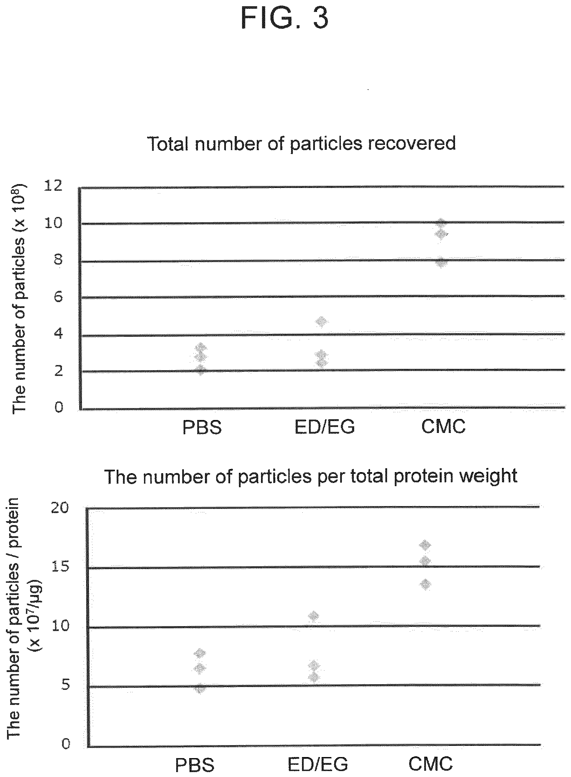

200 µL of healthy human serum was centrifuged at 20,000×g at 4°C for 15 minutes, then the resultant supernatant was diluted with 200 µL of PBS, EDTA/EGTA-PBS (PBS containing EDTA and EGTA with a final concentration of 50 mM after the dilution of the serum, respectively) (ED/EG), or CMC-PBS (PBS in which CMC was dissolved to have a final concentration of 0.5% by weight after the dilution of the serum) (CMC). Then, the magnetic particles (Dynabeads M-280 tosylactivated (Life Technologies)) on which the anti-CD9 antibody (our product) was immobilized were added to the resultant solution to achieve 0.26 mg/mL. After the resultant solution underwent reaction while stirred at 37°C for 30 minutes, the magnetic particles were separated magnetically from the remaining solution (B/F separation). The separated magnetic particles were washed three times with PBS-T, then reacted with 40 µL of Britton & Robinson Universal Buffer (BRUB) (pH 2.6) for 5 minutes, and then neutralized with 20 µL of 1M Tris-HCl (pH 8.0) to separate the extracellular vesicles from the antibody magnetic particles. After total protein concentration was determined by Qubit protein assay (Life Technologies), 450 µL of PBS was added for use in the analysis of the number of particles using NanoSight (FIG. 3). The increase in the total number of recovered particles of EV and the number of particles for total amount of protein were recognized under the dilution of CMC, demonstrating that the extracellular vesicles was recovered at a high purity.

### (Example 4)

The effect on EV recovery of CMC (Sigma-Aldrich Co. LLC #C4888) was investigated for the serum and a plasma that contains each of five kinds of anticoagulants (heparin, EDTA, citrate, acid-citrate-dextrose (ACD) and citrate-phosphate-dextrose (CPD)).

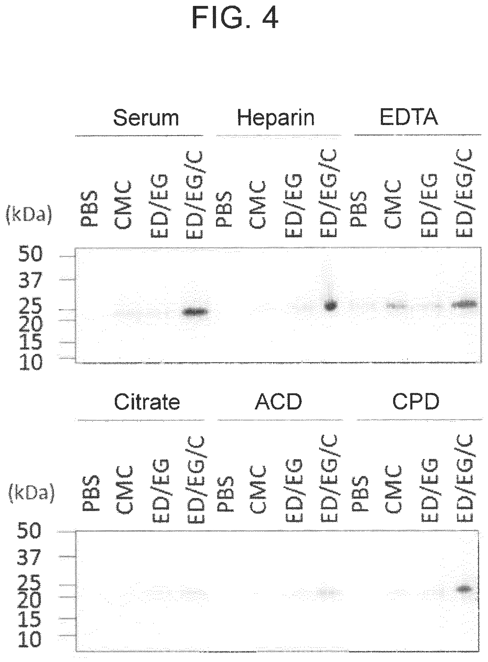

200 µL of the healthy human serum and the anticoagulant-containing healthy human plasma was centrifuged at 20,000×g at 4°C for 15 minutes, then the resultant supernatant was diluted with 200 µL of PBS, EDTA/EGTA-PBS (PBS containing EDTA and EGTA with a final concentration of 50 mM after the dilution of the serum or plasma, respectively) (ED/EG), or CMC-PBS (PBS in which CMC was dissolved to have a final concentration of 0.5% by weight after the dilution of the serum or plasma) (CMC), or EDTA/EGTA/CMC-PBS (PBS that contains EDTA, EGTA and CMC with a final concentration of 37.5 mM/37.5 mM/0.5% by weight after the dilution of the serum or plasma) (ED/EG/C). Then, magnetic particles (Dynabeads M-280 tosylactivated (Life Technologies)) on which the anti-CD9 antibody (our product) was immobilized were added to the resultant solution to achieve 0.26 mg/mL. After the resultant solution underwent reaction while stirred at 37°C for 1 hour, the magnetic particles were separated magnetically from the remaining solution (B/F separation). The separated magnetic particles were washed three times with PBS-T, then diluted with the sample buffer (Bio-Rad Laboratories, Inc.) for preparation of the western blotting sample. Immunoprecipitation efficiency was analyzed by western blotting method with use of the biotinylated anti-CD9 antibody (FIG. 4). Improvement of EV recovery efficiency using CMC was recognized also for the plasma specimen regardless of kinds of the anticoagulants. The combination use of CMC and the chelating agent enabled to further improve the EV recovery efficiency.

### (Example 5)

The effect on EV recovery of CMC (Sigma-Aldrich Co. LLC #C4888) using antibodies against two kinds of tetraspanin membrane proteins (CD63 and CD81) other than CD9 and against an extracellular matrix metalloproteinase inducer (CD147), was investigated.

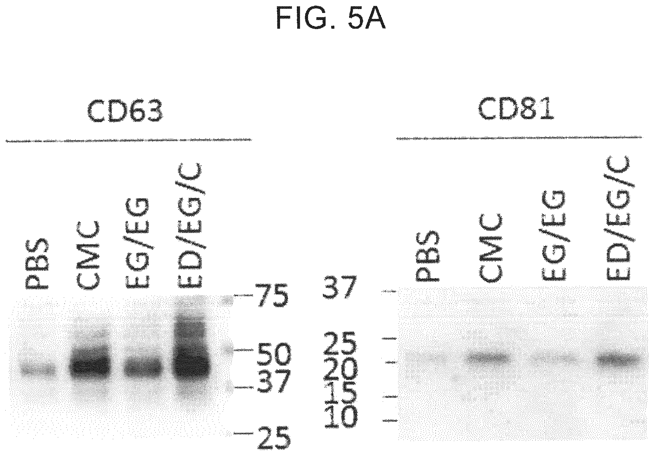

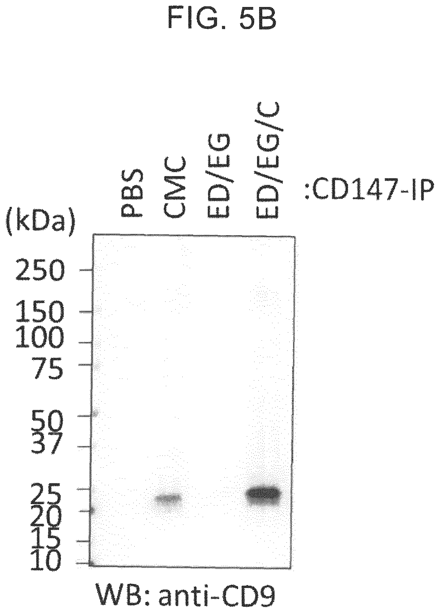

200 µL of the healthy human serum was centrifuged at 20,000×g at 4°C for 15 minutes, then the resultant supernatant was diluted with 200 µL of PBS, EDTA/EGTA-PBS (with a final concentration of 50 mM after the dilution of the serum, respectively) (ED/EG), CMC-PBS (with a final concentration of 0.5% by weight after the dilution of the serum) (CMC), or EDTA/EGTA/CMC-PBS (with final concentrations of 37.5 mM/37.5 mM/0.5% by weight after the dilution of the serum, respectively) (ED/EG/C). Then, the magnetic particles (Dynabeads M-280 tosylactivated (Life Technologies)) on which the anti-CD63 antibody (8A12: Cosmo Bio Co., Ltd.), the anti-CD81 antibody (M38: Abcam) or the anti-CD147 antibody (MEM-M6/1: Abeam) was immobilized were added to the resultant solution to achieve 0.26 mg/mL. After the resultant solution underwent reaction at 4°C while stirred overnight, the magnetic particles were separated magnetically from the remaining solution (B/F separation). The separated magnetic particles were washed three times with PBS-T, then diluted with a sample buffer (Bio-Rad Laboratories, Inc.) for preparation of a western blotting sample. The EV recovery efficiency was analyzed by western blotting method with use of the anti-CD63 antibody (our product), the anti-CD81 antibody (12C4: Cosmo Bio Co., Ltd.) and the biotinylated anti-CD9 antibody (our product) (FIG. 5A and FIG. 5B). Improvement of EV recovery efficiency using CMC was recognized also in the case of using the antibodies against CD63, CD81 and CD147. The combination use of CMC and the chelating agent enabled to further improve the EV recovery efficiency.

### (Example 6)

The effect on EV recovery of CMC (Sigma-Aldrich Co. LLC #C4888) using two kinds of body fluid (urea and saliva) was investigated.

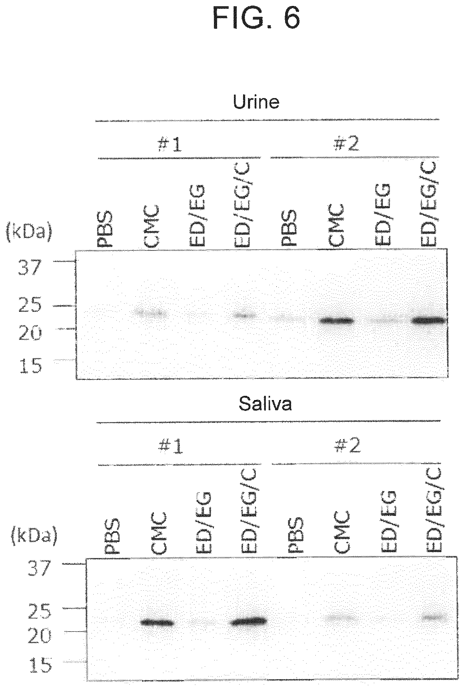

200 uL of the healthy human urea or saliva (two samples that are referred to as "#1" and "#2", respectively) was centrifuged at 15,000×g at 4°C for 15 minutes. After filtered through 0.22 µm filter, the resultant solution was diluted with 200 µL of PBS, EDTA/EGTA-PBS (with a final concentration of 50 mM after the dilution of urine or saliva) (ED/EG), CMC-PBS (with a final concentration of 0.5% by weight after the dilution of urine or saliva) (CMC), or EDTA/EGTA/CMC-PBS (with final concentrations of 37.5 mM/37.5 mM/0.5% by weight after the dilution of urine or saliva, respectively) (ED/EG/C). Then, the magnetic particles (Dynabeads M-280 tosylactivated (Life Technologies)) on which the anti-CD9 antibody (our product) was immobilized were added to the resultant solution to achieve 0.26 mg/mL. After the resultant solution underwent reaction while stirred at 37°C for 1 hour, the magnetic particles were separated magnetically from the remaining solution (B/F separation). The separated magnetic particles were washed three times with PBS-T, then diluted with the sample buffer (Bio-Rad Laboratories, Inc.) for preparation of the western blotting sample. Immunoprecipitation efficiency was analyzed by western blotting method with use of the biotinylated anti-CD9 antibody (FIG. 6). Improvement of EV recovery efficiency using CMC was recognized also for urine and saliva as well as serum and plasma.

### (Example 7)

The effect on EV recovery of cellulose derivatives (with a final concentration of 0.13% by weight to 4.0% by weight) listed in Table 2 below.

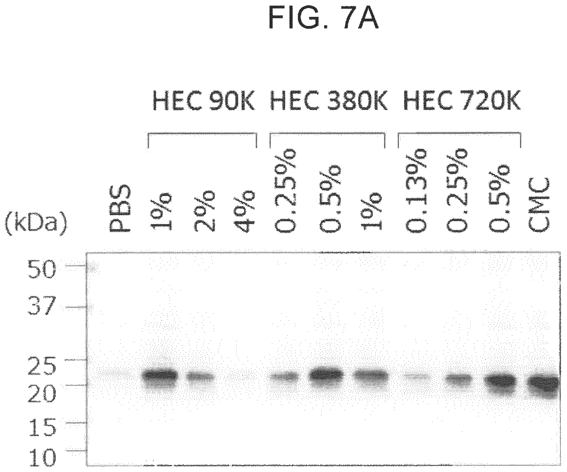

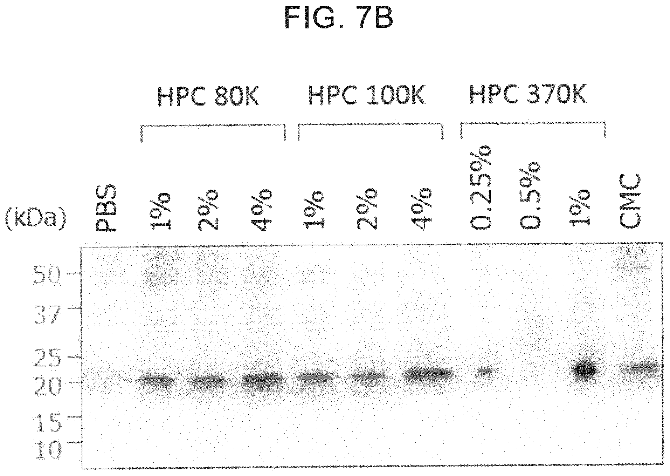

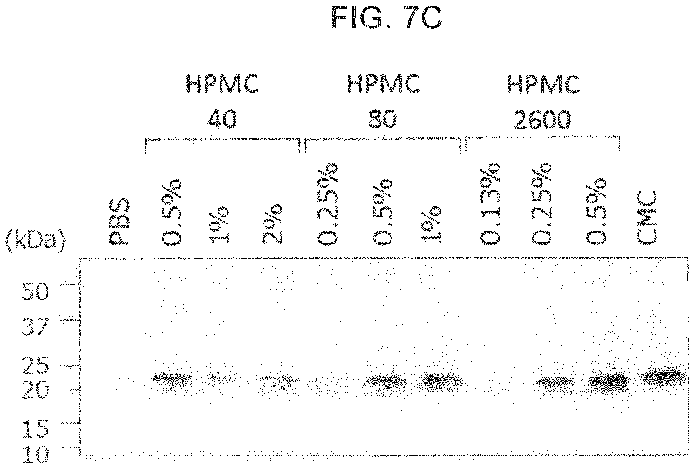

The healthy human serum was centrifuged at 20,000×g at 4°C for 15 minutes, then 200 µL of the serum was diluted with 200 µL of PBS, CMC-PBS (with a final concentration of 0.5% by weight after the dilution of the serum) (CMC), or cellulose derivatives (with a final concentration of 0.13 to 4.0% by weight after the dilution of the serum) dissolved in PBS. Then, the magnetic particles (Dynabeads M-280 tosylactivated (Life Technologies)) on which the anti-CD9 antibody (our product) was immobilized were added to the resultant solution to achieve 0.26 mg/mL. After the resultant solution underwent reaction while stirred at 37°C for 1 hour, the magnetic particles were separated magnetically from the remaining solution (B/F separation). The separated magnetic particles were washed three times with PBS-T, then diluted with the sample buffer (Bio-Rad Laboratories, Inc.) for preparation of the western blotting sample. The EV recovery efficiency was analyzed by western blotting method with use of the biotinylated anti-CD9 antibody (FIGS. 7A to 7C). Improvement of EV recovery efficiency was recognized also in the case of using three kinds of cellulose derivative other than CMC, compared to the PBS-diluted samples (0.13% by weight to 2.0% by weight for HEC, 0.25% by weight to 4.0% by weight for HPC, and 0.25% by weight to 2.0% by weight for HPMC).

**Table 2. List of cellulose derivative used in Example (Manufacturer is Sigma-Aldrich Co. LLC for all)**

| Cellulose derivative | Product name | Average molecular weight (kDa) | Viscosity / Measurement condition (Value disclosed by Manufacturer) |
|---|---|---|---|
| Hydroxyethyl cellulose (HEC) CAS No. 9004-62-0 | HEC 90kDa #434965 | Average Mv ∼90 | 75-150 mPa·s/ 5 wt% aqueous solution (25°C) (lit.) |
| | HEC 380kDa #308633 | Average Mv ∼380 | 300-400 mPa·s/ 2 wt% aqueous solution (25°C) |
| | HEC 720kDa #434973 | Average Mv ∼720 | 4, 500-6, 500 mPa·s/ 2 wt% aqueous solution (25°C) (lit.) |
| Hydroxypropyl cellulose (HPC) CAS No. 9004-64-2 | HPC 80kDa #435007 | Average Mn ∼10 Average Mw ∼80 | 250-800 mPa·s/ 10 wt% aqueous solution (25°C) (lit.) |
| | HPC 100kDa #191884 | Average Mw ∼100 | 75-150 mPa·s/ 5 wt% aqueous solution (25°C) (lit.) |
| | HPC 370kDa #191892 | Average Mw ∼370 | 150-400 mPa·s/ 2 wt% aqueous solution (25°C) (lit.) |
| Hydroxypropyl methylcellulose (HPMC) CAS No. 9004-65-3 | HPMC 40-60cP #H8384 | Average Mw ∼22 | 40-60 mPa·s/ 2 wt% aqueous solution (20°C) (lit.) |
| | HPMC 80-120cP #H9262 | Average Mw ∼26 | 80-120 mPa·s/ 2 wt% aqueous solution (20°C) (lit.) |
| | HPMC 2600-5600cP #H7509 | Average Mw ∼86 | 2,600-5,600 mPa· s/2 wt% aqueous solution (20°C) (lit.) |

### (Example 8)

The effect of polyvinylpyrrolidone (with final concentrations of 1% by weight, 2% by weight and 4% by weight) listed in Table 3 below on the EV recovery was investigated.

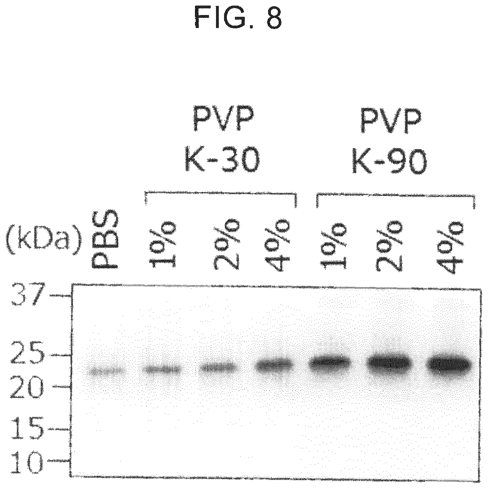

The healthy human serum was centrifuged at 20,000×g at 4°C for 15 minutes, then 200 µL of the serum was diluted with 200 µL of PBS, or polyvinylpyrrolidone (with a final concentration of 1.0 to 4.0% by weight after the dilution of the serum) dissolved in PBS. Then, the magnetic particles (Dynabeads M-280 tosylactivated (Life Technologies)) on which the anti-CD9 antibody (our product) was immobilized were added to the resultant solution to achieve 0.26 mg/mL. After the resultant solution underwent reaction while stirred at 37°C for 1 hour, the magnetic particles were separated magnetically from the remaining solution (B/F separation). The separated magnetic particles were washed three times with PBS-T, then diluted with the sample buffer (Bio-Rad Laboratories, Inc.) for preparation of the western blotting sample. Recovery efficiency of EV was analyzed by western blotting method with use of the biotinylated anti-CD9 antibody (FIG. 8). Improvement of EV recovery efficiency was recognized for polyvinylpyrrolidone in a range of 1.0% by weight to 4.0% by weight, compared to the PBS-diluted samples.

**Table 3. List of polyvinylpyrrolidone (PVP) used in Example**

| Polyvinyl derivative | Product name | Manufacturer | Average molecular weight (kDa) |
|---|---|---|---|
| Polyvinylpyrrolidone (PVP) CAS No. 9003-39-8 | PVP K-30 28314-82 nacalai | Nacalai Tesque, Inc. | Mw 40 |
| | PVP K-90 28315-72 nacalai | Nacalai Tesque, Inc. | Mw 360 |

### (Example 9)

The effect on the EV recovery was investigated at different reaction temperatures ranging from 35°C to 60°C for CMC (Sigma-Aldrich Co. LLC #C4888).

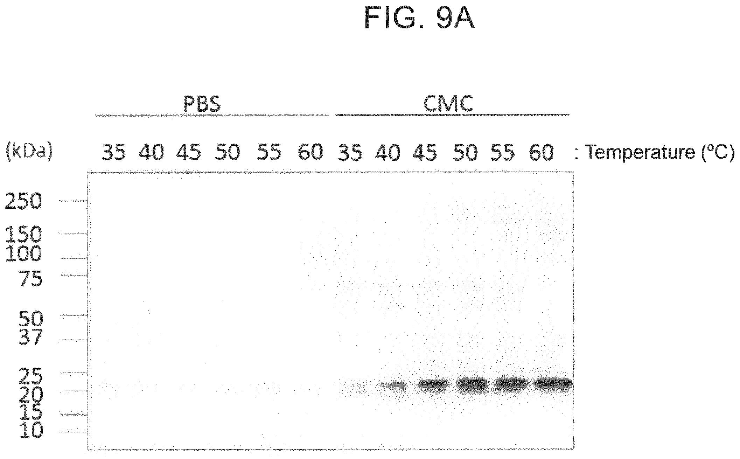

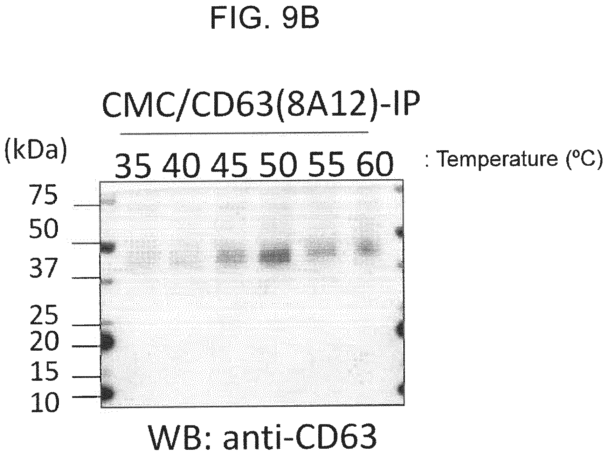

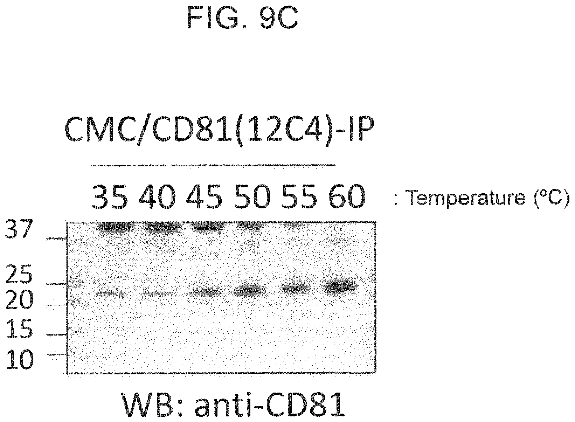

The healthy human serum was centrifuged at 20,000×g at 4°C for 15 minutes, then 100 µL of the serum was diluted with 100 µL of PBS, or CMC-PBS (with a final concentration of 0.5% by weight after the dilution of the serum). Then, the magnetic particles (Dynabeads M-280 tosylactivated (Life Technologies)) on which the anti-CD9 antibody (our product), the anti-CD63 antibody (8A12: Cosmo Bio Co., Ltd.) or the anti-CD81 antibody (12C4: Cosmo Bio Co., Ltd.) was immobilized were added to the resultant solution to achieve 0.26 mg/mL. After the reaction at each temperature for 5 minutes, the magnetic particles were separated by magnetic collection from the remaining solution (B/F separation). The separated magnetic particles were washed three times with PBS-T, then diluted with the sample buffer (Bio-Rad Laboratories, Inc.) for preparation of the western blotting sample. The EV recovery efficiency was analyzed by western blotting method with use of the biotinylated anti-CD9 antibody (our product), anti-CD63 antibody (our product), and anti-CD81 antibody (12C4: Cosmo Bio Co., Ltd.) (FIG. 9A to FIG. 9C). Improvement of EV recovery efficiency was recognized at each temperature ranging from 35°C to 60°C with the addition of CMC. Further improvement of EV recovery efficiency was recognized at high temperatures of 40°C or more with the addition of CMC. The improvement of EV recovery efficiency using CMC can be recognized also for short-time reactions.

### (Example 10)

Viscosities of the cellulose derivatives used in Example 7 and polyvinylpyrrolidone used in Example 8 in the PBS solutions were measured. Specifically, the viscosity was obtained as an average of results that were measured at 30°C for 60 seconds at different rotation speeds of 200 rpm, 400 rpm, 600 rpm and 800 rpm with use of a viscosity analyzer, Rheology Spectrometer SKR100 (Yamato Scientific Co., Ltd.), for each of PBS solutions that were prepared by dissolving each of the cellulose derivatives or polyvinylpyrrolidone in PBS to achieve 2% by weight (Table 4) .

**Table 4. Viscosities of cellulose derivative and polyvinylpyrrolidone used in Example**

| | Product name | Viscosity / Measurement condition |
|---|---|---|
| Hydroxyethyl cellulose (HEC) CAS No. 9004-62-0 | HEC 380kDa #308633 | 207.8 mPa·s/ 2 wt% PBS solution (30°C) |
| Hydroxypropylmethyl cellulose (HPMC) CAS No. 9004-65-3 | HPMC 80-120cP #H9262 | 48.6 mPa·s/ 2 wt% PBS solution (30°C) |
| Polyvinylpyrrolidone (PVP) CAS No. 9003-39-8 | PVP K-30 28314-82 nacalai | 1.5 mPa·s/ 2 wt% PBS solution (30°C) |
| | PVP K-90 28315-72 nacalai | 5.8 mPa·s/ 2 wt% PBS solution (30°C) |

### (Example 11)

The effect on the EV recovery by means of immunoprecipitation using CMC (Sigma-Aldrich Co. LLC #C4888) and antibodies against tetraspanin membrane proteins (CD9 and CD63) and detection of marker (EML4-ALK fusion gene) RNA from the EV, were investigated.

A culture supernatant of human lung carcinoma cell line H2228 cultured in serum-free medium for three days was used as a sample. The culture supernatant was centrifuged at 2,000×g at 4°C for 5 minutes, then filtered through a 0.22 µm filter (manufactured by Millipore Corp.), and then concentrated using Amicon Ultra-15 (manufactured by Millipore Corp.) to 100-fold.

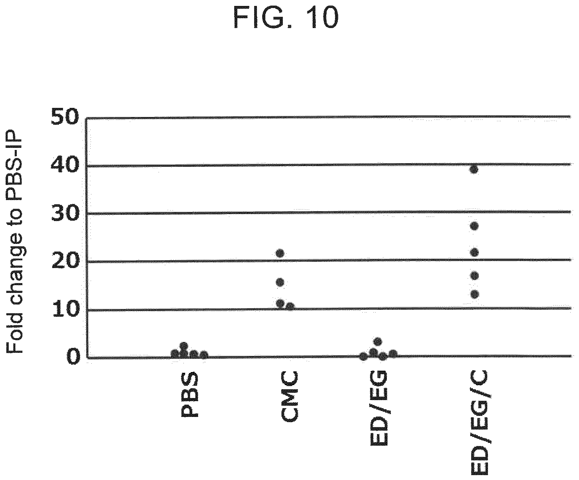

The concentrate was diluted with an equivalent amount of PBS, EDTA/EGTA-PBS (with a final concentration of 50 mM after the dilution of the concentrate, respectively) (ED/EG), CMC-PBS (with a final concentration of 0.5%by weight after the dilution of the concentrate) (CMC), or EDTA/EGTA/CMC-PBS (with final concentrations of 37.5 mM/37.5 mM/0.5% by weight after the dilution of the concentrate, respectively) (ED/EG/C). Then, Dynabeads M-280 tosylactivated (Life Technologies) on which the anti-CD9 antibody (our product) or the anti-CD63 antibody (our product) was immobilized were added to the resultant solution to achieve 0.26 mg/mL. After the resultant solution underwent reaction while stirred at 4°C overnight, the magnetic particles were separated by magnetic collection from the remaining solution (B/F separation). The separated magnetic particles were washed three times with PBS-T, then total RNA was purified using miRNeasy micro kit (manufactured by QIAGEN). cDNA was prepared from the purified total RNA using Superscript (trademark) IV First-Strand Synthesis System (manufactured by Thermo Fisher Scientific), and then EML4-ALK mRNA was quantified by using Droplet Digital PCR (Bio-Rad Laboratories, Inc.) (FIG. 10).

For the detection of EML4-ALK mRNA, primers and a fluorescence probe with sequences listed in Table 5 below were used. As the fluorescence probe, a double quencher probe having a fluorescent substance HEX at 5'-terminal and ZEN quencher in the probe and Iowa Black (registered trademark) quencher (IABkFQ) at 3'-terminal, was used. With use of the primers and fluorescence probes listed in Table 5, it is possible to detect variants 3a and 3b of the EML4-ALK fusion gene.

**Table 5. Probe and primers for detecting EML4-ALK mRNA**

| Primer/Probe | Sequence |
|---|---|
| Forward primer | CAGATGATAGCCGTAATAAATTGTCG (SEQ ID NO: 1) |
| Reverse primer | CTTCCGGCGGTACACTTGG (SEQ ID NO: 2) |
| Fluorescence probe | /5HEX/ACTGCAGAC/ZEN/AAGCATAAAGATGTCA (SEQ ID NO: 3)/3IABkFQ/ |

EML4-ALK contained in EV that was collected by immunoprecipitation, was detected. The use of CMC increased the amount of EML4-ALK mRNA detected, revealing improvement of the EV recovery efficiency. Furthermore, the addition of the chelating agent further increased the amount of EML4-ALK mRNA detected, revealing the EV recovery efficiency was further improved.

### (Example 12)

### Investigation of ultrasonic effect on magnetic collection efficiency of magnetic particles (1)

The effect of ultrasonication on the magnetic collection efficiency of the magnetic particles was investigated by applying ultrasonication (in a water tank) after the reaction of the extracellular vesicles (EVs) with the antibody-bound magnetic particles in the presence of carboxymethylcellulose (CMC).

35.4 ng of EV collected from DU145 (human prostate cancer cell line) and the magnetic particles with 1.2 mg/mL final concentration (Dynabeads M-280 tosylactivated (Life Technologies #14204)) on which the anti-CD9 antibody was immobilized were added to 600 µL of EDTA/EGTA/CMC-PBS solution (with final concentrations of 50 mM/50 mM/0.5% by weight, respectively) in a 2 mL tube. After the resultant solution underwent reaction for one hour at 37°C, ultrasonication was applied to the tube at different frequencies of 100 kHz (50w), 200 kHz (100w), 950 kHz (100w), and 1.6 MHz (100w) for 3 minutes with use of an ultrasonic generator device (water bath type) (KAIJO corporation, QUAVAmini QR-001 and QR-003). As negative control, another solution was prepared without the application. Another solution was prepared by adding cellulase (Cellulase from Aspergillus sp. SIGMA #C2605-50ML) at 1/150 of the solution amount and then reacted at 37°C for 5 minutes, instead of application of ultrasonication. A water bath type transducer was used as the transducer of the ultrasonic device. In each condition, after the ultrasonic application, the magnetic particles were separated magnetically from the remaining solution (B/F separation). The solution obtained after the separation was collected. The magnetic particle content was investigated in terms of a value measured with a spectrophotometer (wavelength: 500 nm) (JASCO Corporation, V-650). The CMC used was #C4888 (Sigma-Aldrich) with an average molecular weight of 250 kDa.

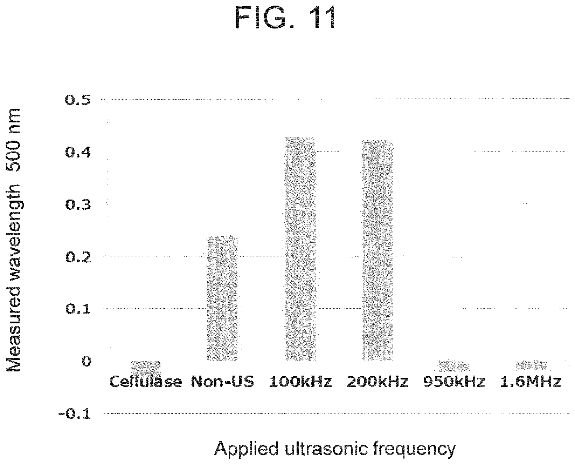

The results revealed that the application at 950 kHz or more substantially decreased the amount of the magnetic particles contained in the solution after the B/F separation, compared to the case without application. This demonstrates that the efficiency of the magnetic collection was significantly improved to achieve a high magnetic collection efficiency by the application at 950 kHz or more (FIG. 11). As well, the addition of cellulase was revealed to achieve the high magnetic collection efficiency.

### (Example 13)

### Investigation of ultrasonic effect on magnetic collection efficiency of magnetic particles (2)

The effect of ultrasonication on the EV recovery resulting from the improvement of magnetic collection efficiency was investigated by application of ultrasonication after the reaction of the EVs with the antibody-bound particles in the presence of CMC.

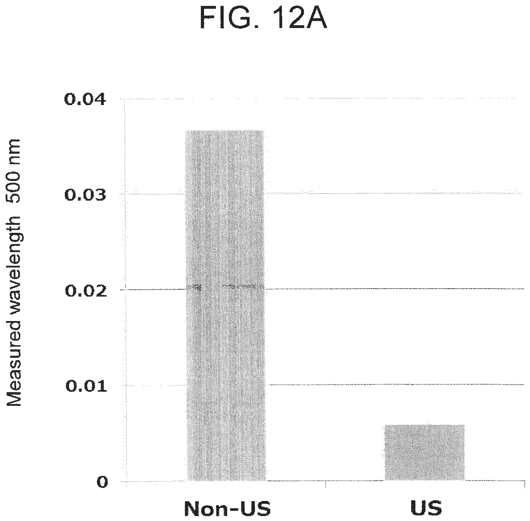

1.3 pg of EV collected from BxPC3 (human pancreatic adenocarcinoma cell line) and 1.2 mg/mL final concentration of Dynabeads M-280 tosylactivated (Life Technologies #14204) on which the anti-CD9 antibody was immobilized were added to 600 µL of EDTA/EGTA/CMC-PBS solution (with final concentrations of 50 mM/50 mM/0.5% by weight, respectively) in the 2 mL tube. After the resultant solution underwent reaction for one hour at 37°C, ultrasonication was applied at a frequency of 30 kHz (35 w) for 3 minutes with use of QUAVAmini QR-001 (KAIJO corporation). As negative control, another solution was prepared without application. A dry type transducer was used as the transducer of the ultrasonic device. After the ultrasonic application, the magnetic particles were separated magnetically from the remaining solution (B/F separation). The solution obtained after the separation was collected (collected supernatant). The magnetic particle content in the collected supernatant was investigated by comparison in terms of the value measured with the spectrophotometer (wavelength: 500 nm) (JASCO Corporation, V-650) (FIG. 12A).

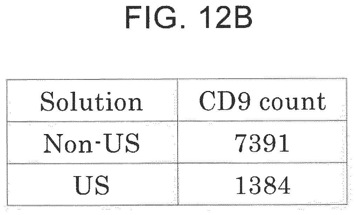

The content of CD9 in the collected solution was investigated by sandwich CLEIA method with use of an anti-CD9 antibody (FIG. 12B).

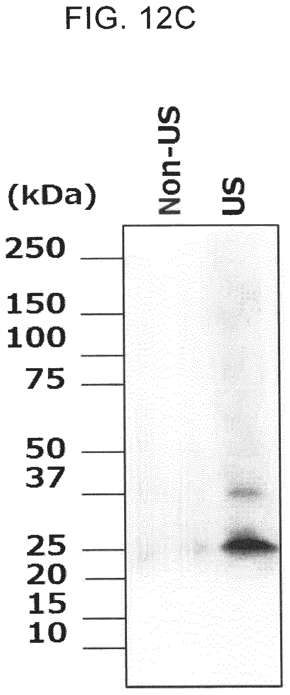

The EV recovery efficiency was investigated by Western blotting detection (25 kDa) for the magnetic particles obtained after B/F separation with an anti-CD9 antibody (Fig. 12C).

Specifically, the sandwich CLEIA method using the above anti-CD9 antibody was performed by the following method. First, the collected solution was mixed with Dynabeads on which the anti-CD9 antibody was immobilized and the resulting mixture solution was incubated at 37°C for 8 minutes. After the B/F separation and washing, 50 µL of alkaline Phosphatase-labeled anti-CD9 antibody was added, then the resulting solution was stirred and then incubated at 37°C for 8 minutes, and B/F separation and washing were carried out. Then, 200 µL of Lumipulse (registered trademark) substrate solution (Fujirebio Inc.) containing 3-(2'-spiroadamantane)-4-methoxy-4-(3"-phosphoryloxy)phenyl-1,2-dioxetane disodium salt serving as a chemiluminescent substance, was dispensed. After stirred, the resulting solution was incubated at 37°C for 4 minutes and analyzed by measurement with luminometer to generate a luminescence intensity. A count value was obtained as the measured value. The luminescence intensity was measured using a fully automated chemiluminescent enzyme immunoassay system (Lumipulse L2400 (Fujirebio Inc.)). The lower amount of CD9 in the collected solution (the smaller the CD9 count value) can be interpreted as more efficient EV recovery.

The results revealed that the ultrasonication-applied sample was improved in terms of the magnetic collection efficiency and the EV recovery efficiency, compared to the sample not subjected to ultrasonication-applied (Figures 12A to 12C).

### (Example 14)

### Investigation of influence of CMC concentration on magnetic collection efficiency of magnetic particles

The influence of CMC concentration on magnetic collection efficiency of the magnetic particles was investigated.

1.3 µg of EV collected from BxPC3 (human pancreatic adenocarcinoma cell line) and 1.2 mg/mL final concentration of Dynabeads M-280 tosylactivated (Life Technologies #14204)) on which the anti-CD9 antibody was immobilized were added to 600 µL of EDTA/EGTA/CMC-PBS solution (with final concentrations of 50 mM/50 mM/1%, 0.5% or 0.25% by weight, respectively) with each of three different CMC concentrations 1% by weight, 0.5% by weight and 0.25% by weight. After the mixture underwent reaction for binding at 37°C for one hour, ultrasonication was applied at 30 kHz (35 w) for 3 minutes with use of QUAVAmini QR-001 (KAIJO corporation). As negative control, another solution was prepared using the EDTA/EGTA/CMC-PBS solution with CMC concentration of 0.5% by weight without ultrasonic application. A dry type transducer was used as the transducer of the ultrasonic device. After the ultrasonic application, the magnetic particles were separated magnetically from the remaining solution (B/F separation). The solution obtained after the separation was collected. The magnetic particle content was compared in terms of the value measured with the spectrophotometer (wavelength: 500 nm) (JASCO Corporation, V-650).

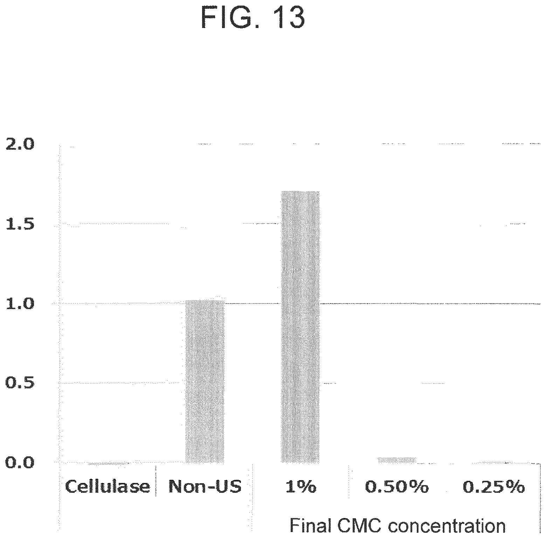

The results revealed that the magnetic collection efficiency was improved in the ultrasonic-applied samples of the EDTA/EGTA/CMC-PBS solution with CMC concentration of 0.5% by weight or less, compared to the solution not subjected to ultrasonic application (FIG. 13).

### (Example 15)

### Investigation of ultrasonic effect on magnetic collection efficiency of magnetic particles in solution containing cellulose derivative other than CMC

The ultrasonic effect on the magnetic collection efficiency of the magnetic particles in solutions containing a cellulose derivative other than CMC was investigated by comparison.

1.3 pg of EV collected from BxPC3 (human pancreatic adenocarcinoma cell line) and 1.2 mg/mL final concentration of Dynabeads M-280 tosylactivated (Life Technologies #14204)) on which the anti-CD9 antibody was immobilized were added to 600 µL of the cellulose derivative solution (0.25% by weight to 4.0% by weight) obtained by dissolving the cellulose derivative into PBS. After undergoing reaction for binding at 37°C for one hour, the resulting solution was subjected to the application at 30 kHz (35 w) for 3 minutes with use of QUAVAmini QR-001 (KAIJO corporation). As negative control, another solution was prepared using the EDTA/EGTA/CMC-PBS solution with CMC concentration of 0.5% by weight without application. A dry type transducer was used as the transducer of the ultrasonic device. After the ultrasonic application, the magnetic particles were separated magnetically from the remaining solution (B/F separation). The solution obtained after the separation was collected. The magnetic particle content was compared in terms of the value measured with the spectrophotometer (wavelength: 500 nm) (JASCO Corporation, V-650). The cellulose derivatives used were hydroxypropyl cellulose (HPC): average molecular weight 80 kDa #435007 and hydroxyethyl cellulose (HEC): average molecular weight 380 kDa #308633 (both Sigma-Aldrich).

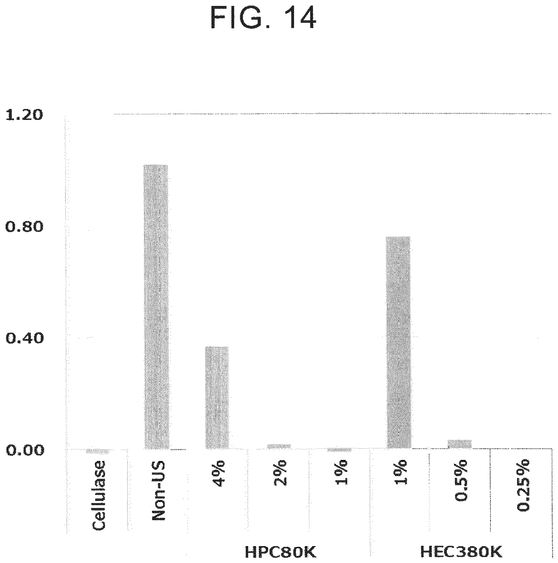

The results revealed that the magnetic collection efficiency was improved also in the ultrasonic-applied samples of the solutions containing the cellulose derivative other than CMC, compared to the solution not subjected to ultrasonic application. The effect was particularly pronounced at 2.0% by weight or less for HPC and 0.5% by weight or less for HEC (FIG. 14).

### (Example 16)

### Investigation of influence of ultrasonic output on magnetic collection efficiency of magnetic particles

The influence of ultrasonic output on the magnetic collection efficiency of the magnetic particles was investigated.

1.3 µg of EV collected from BxPC3 (human pancreatic adenocarcinoma cell line) and 1.2 mg/mL final concentration of Dynabeads M-280 tosylactivated (Life Technologies #14204)) on which the anti-CD9 antibody was immobilized were added to 600 µL of EDTA/EGTA/CMC-PBS solution (with final concentrations of 50 mM/50 mM/0.5% by weight, respectively) in the 2 mL tube. After undergoing reaction for binding at 37°C for one hour, the resulting solution was subjected to the application at 30 kHz (output: 10 w, 20w or 35 w) for 3 minutes with use of QUAVAmini QR-001 (KAIJO corporation). The application was performed also with a water tank type (frequency 40 kHz, output 70 w, EMERSON BRANSONIC M1800-J) instead of the dry ultrasonic device. As negative control, another solution was prepared without application. After the ultrasonic application, the magnetic particles were separated magnetically from the remaining solution (B/F separation). The solution obtained after the separation was collected. The magnetic particle content was compared in terms of the value measured with the spectrophotometer (wavelength: 500 nm) (JASCO Corporation, V-650).

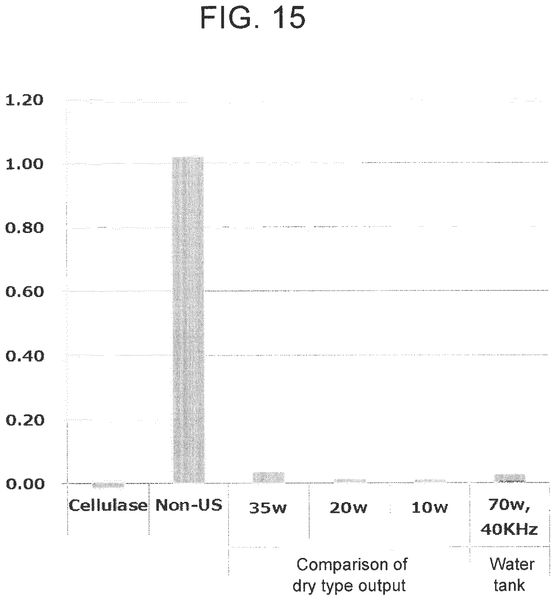

The results revealed that the magnetic collection efficiency was improved in the ultrasonic-applied sample under all output conditions, compared to the solution not subjected to ultrasonic application (FIG. 15).

### (Example 17)

### Investigation of effect of sugar degrading enzyme on EV recovery efficiency

It was investigated whether a sugar degrading enzyme improves the EV recovery efficiency in the cellulose derivative-containing solution.

200 uL of healthy human serum was centrifuged at 20,000×g at 4°C for 15 minutes, then diluted with 200 µL of EDTA/EGTA/CMC-PBS (with final concentrations of 37.5 mM/37.5 mM/0.5% by weight, respectively). Then, Dynabeads M-280 tosylactivated (Life Technologies) on which the anti-CD9 antibody was immobilized were added to the resultant solution to achieve 0.26 mg/mL. After the resultant solution underwent reaction while stirred at 4°C overnight, endoglucanase was added for degrading CMC to decrease a viscosity of the solution. Then, the magnetic particles were collected from the solution (B/F separation) and the magnetic particles were washed three times with PBS-T at 37°C. Next, the magnetic particles were treated with the sample buffer (Bio-Rad Laboratories, Inc.) for preparation of the western blotting sample and the EV recovery efficiency was analyzed by western blotting method with use of the anti-CD9 antibody.

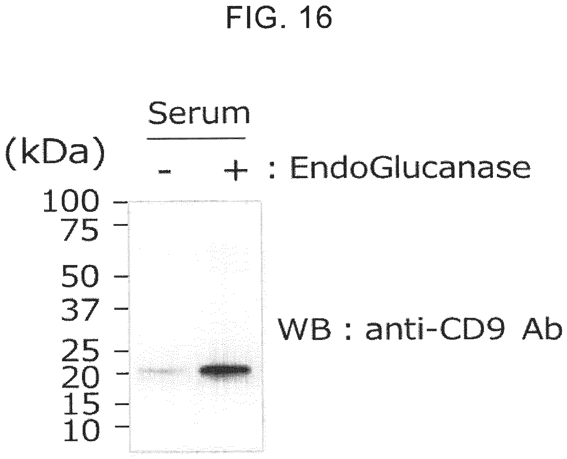

The results revealed that the sugar degrading enzyme improves the EV recovery efficiency (FIG. 16). The sugar degrading enzyme was considered to improve the EV recovery efficiency by the improvement of the magnetic collection efficiency of the magnetic particles.

### (Example 18)

### Investigation of effect of specimen type on EV recovery efficiency

It was investigated whether the magnetic collection efficiency can be improved by ultrasonication also in a serum specimen.

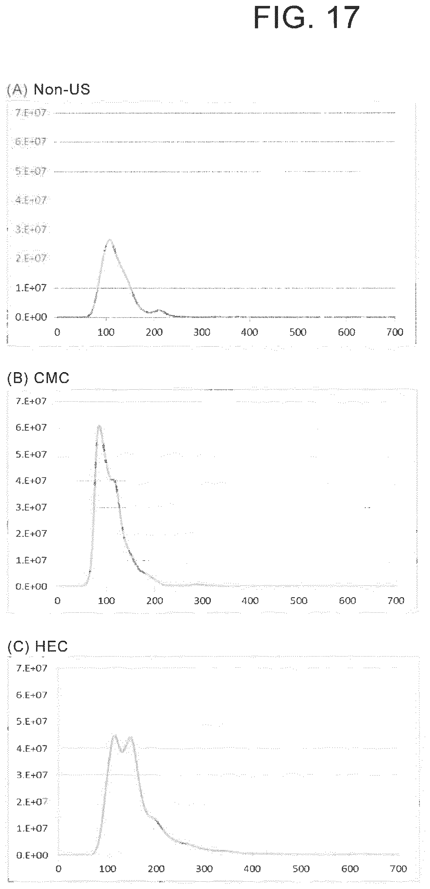

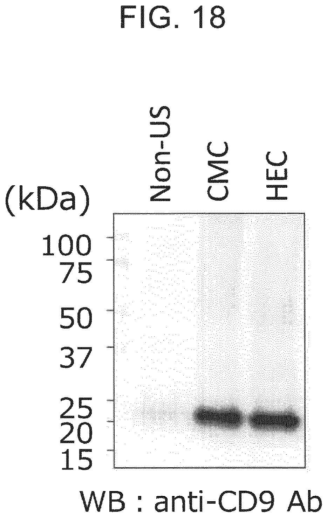

To the tube, 300 µL of serum specimen, CMC (with final concentration of 0.5% by weight) and 300 µL of HEC (solution with a final concentration of 0.25% by weight) were added. To this mixture, 1.2 mg/mL final concentration of Dynabeads M-280 tosylactivated (Life Technologies, #14204) on which the anti-CD9 antibody was immobilized was added. The resultant solution underwent reaction while stirred at 4°C overnight, and then subjected to application at 30 kHz (35 w) for 3 minutes with use of QUAVAmini QR-001 (KAIJO corporation). A negative control was prepared without application under the condition of CMC (with a final concentration of 0.5% by weight). After applying ultrasonication, the magnetic particles were separated magnetically from the remaining solution (B/F separation). The solution obtained after the separation was collected. The magnetic particle content was compared in terms of the value measured with the spectrophotometer (wavelength: 500 nm) (JASCO Corporation, V-650) (Table 6, Magnetic particle contents). The magnetic particles in the tube obtained after separated from the solution were washed three times with PBS-T, then separated into two groups. One was reacted with 40 µL BRUB (Britton & Robinson Universal Buffer) (pH 2.6) for 5 minutes, then neutralized with 20 µL 1M Tris-HCl (pH 8.0) to release the extracellular vesicles from the antibody particles, and then used for nanoparticle tracking analysis (NanoSight LM10, Quantum Design, Inc.) (FIG. 17). The other was treated with sample buffer (BIO-RAD) for the preparation of the Western blotting sample, and used for analysis of the EV recovery efficiency by Western blotting method with anti-CD9 antibody (FIG. 18).

FIG. 17 represents the nanoparticle tracking analysis of extracellular vesicles released from the magnetic particles represented by plotting with particle size as a horizontal axis and number of particles /mL as a vertical axis. Table 6 lists the concentration (number of particles /mL) of the extracellular vesicles released from the magnetic particles, calculated from the results represented in FIG. 17. The results reveals that the magnetic collection efficiency and EV recovery efficiency were improved by ultrasonic treatment also in the serum sample, compared to the solution not subjected to ultrasonic treatment (Table 6, FIGS. 17 and 18).

**Table 6. Assessment of incorporation of magnetic particles and concentration of extracellular vesicle recovered in the case of using serum specimen**

| | Magnetic particle incorporation degree OD | Number of particles/mL |
|---|---|---|
| Without ultrasonic treatment | 1.5102 | 1.67x10⁹ |
| Ultrasonic treatment (using CMC) | 1.2072 | 3.41x10⁹ |
| Ultrasonic treatment (using HEC) | 1.2103 | 4.28x10⁹ |

## Claims

1. A method for recovering an extracellular vesicle, the method comprising the following steps (a) and (b):
(a) mixing (i) an extracellular vesicle-containing sample, (ii) particles on which a substance having an affinity to extracellular vesicle membrane is immobilized and (iii) a polymer to give a mixture solution containing (i') target particles bound to the extracellular vesicle via the substance and (ii') the polymer; and
(b) separating the target particles from the mixture solution.

2. The method according to claim 1, comprising the following steps (a) to (c):
(a) mixing (i) the extracellular vesicle-containing sample, (ii) the particles on which the substance having the affinity to the extracellular vesicle membrane is immobilized and (iii) the polymer to give the mixture solution containing (i') the target particles bound to the extracellular vesicle via the substance and (ii') the polymer;
(b) reducing a viscosity of the mixture solution; and
(c) separating the target particles from the solution obtained in the step (b).

3. The method according to claim 1 or 2, comprising (I) washing the target particles and/or (II) releasing the extracellular vesicle from the target particles after separating the target particles.

4. The method according to claim 2 or 3, wherein the viscosity is reduced by a treatment by ultrasonication or with an enzyme capable of degrading a polymer.

5. The method according to claim 4, wherein the ultrasonication is dry type or water bath type.

6. The method according to claim 4 or 5, wherein a frequency of the ultrasonication is in a range of 40 kHz or less or 950 kHz or more.

7. The method according to any one of claims 1 to 6, wherein the polymer is a polysaccharide, a protein, or a polyvinyl derivative having a carbonyl-containing hydrophilic group.

8. The method according to claim 7, wherein the polysaccharide is a cellulose derivative in which a hydrogen atom of at least one hydroxy group in a cellulose is substituted with a carboxyalkyl or hydroxyalkyl.

9. The method according to any one of claims 1 to 8, wherein the polymer has a weight average molecular weight of 10 kDa or more.

10. The method according to any one of claims 1 to 9, wherein a concentration of the polymer in the mixture solution in the step (a) is 0.01 to 10.00% by weight.

11. The method according to any one of claims 1 to 10, wherein (iv) a chelating agent is further mixed in the step (a) .

12. The method according to any one of claims 1 to 11, wherein the substance having the affinity to the extracellular vesicle membrane is an antibody against a tetraspanin membrane protein or an antibody against an extracellular matrix metalloproteinase inducer.

13. The method according to any one of claims 1 to 12, wherein the extracellular vesicle-containing sample is a fluid sample from an animal or a culture supernatant sample.

14. A method for analyzing an extracellular vesicle, the method comprising the following steps (1) and (2):
(1) separating an extracellular vesicle from an extracellular vesicle-containing sample by the method according to any one of claims 1 to 13; and
(2) analyzing the separated extracellular vesicle.

15. A kit comprising (a) a polymer, (b) a substance having an affinity to extracellular vesicle membrane, and (c) an enzyme capable of degrading a polymer,
wherein the substance is in a free form or in a form immobilized on particles, and
the kit optionally further comprises particles when the substance is in a free form.

16. The kit according to claim 15, wherein the polymer is a polysaccharide or a protein and the enzyme capable of degrading the polymer is a sugar degrading enzyme or a proteolytic enzyme.
